# EUROPEAN PATENT APPLICATION

(11) **EP 4 442 839 A1**
(43) Date of publication of application: **09.10.2024**
(21) Application number: 22900297.7
(22) Date of filing: 18.11.2022
(51) Int. Cl.: C12Q 1/6869, C12N 15/11, C40B 40/06

(54) **SEQUENCING LIBRARY CONSTRUCTION METHOD AND APPLICATION**

(30) Priority: 01.12.2021 CN 202111447642
(71) Applicant: Jiangsu Microdiag Biomedical Technology Co., Ltd., Jiangsu, Suzhou Area, 215000 (CN); Suzhou Premed Medical Laboratory Co., Ltd., Suzhou, Jiangsu 215000 (CN)
(72) Inventor: BA, Zhaofen, Suzhou Area, Jiangsu, 215000 (CN); SONG, Ruhui, Suzhou Area, Jiangsu, 215000 (CN); ZHANG, Jiaxin, Suzhou Area, Jiangsu, 215000 (CN); SUN, Hao, Suzhou Area, Jiangsu, 215000 (CN); WANG, Tao, Suzhou Area, Jiangsu, 215000 (CN)
(74) Representative: J A Kemp LLP
(86) International application number: PCT/CN2022/132866
(87) International publication number: WO 2023/098492

(57) **Abstract**

A construction method for a sequencing library, comprising: providing a primer sequence fragment used for performing amplification and library construction on a deoxyribonucleic acid, and processing the primer sequence fragment to obtain an amplification primer with a 5' terminal being G, wherein the processing comprises: if a basic group at the 5' terminal of the primer sequence fragment is not G, G is ligated to the 5' terminal, and if the basic group at the 5' terminal of the primer sequence fragment is G, G is ligated or is not ligated to the 5' terminal; performing cyclic amplification by using the amplification primer with the 5' terminal being G, so as to obtain an amplification fragment with a 3' terminal being C; adding A to the 3' terminal of the deoxyribonucleic acid; and ligating a linker containing a T sticky terminal to the amplification fragment with A being added to the 3' terminal, so as to generate a ligation product. Further disclosed are an amplification primer for constructing a sequencing library, a sequencing library, a kit for constructing a sequencing library, a mutant site detection kit, a chromosome ploidy detection kit, a gene fusion detection kit, and a sequencing method.

## Description

### CROSS-REFERENCE TO RELATED APPLICATIONS

The present application claims priority to Chinese Patent Application No. 2021114476422, entitled "Sequencing library construction method and application", filed on December 01, 2021, the content of which is incorporated herein by reference in its entirety.

### TECHNICAL FIELD

The present application relates to the field of sequencing technology, and specifically to a sequencing library construction method and application.

### BACKGROUND

High-throughput sequencing is also known as massively parallel sequencing or next-generation sequencing. High-throughput sequencing can sequence multiple target regions of one sample or multiple samples at one time, and its use in clinical applications including pharmacogenomics, genetic disease research and screening, tumor mutation gene detection, and clinical microbiological detection has also gradually received attention.

Whole-genome sequencing has made great progress, but there are still problems related to this technology such as incomplete and inaccurate interpretation databases and high sequencing costs. The high cost and technical complexity of this technology limit the application of whole-genome sequencing. To this terminal, various methods of high-throughput sequencing library construction for target region enrichment for specific genomic regions have been widely developed and applied, thereby improving coverage, and achieving the aim of simplifying the process and reducing costs.

There are two main methods of library construction for targeted enrichment of specific regions: probe hybridization capture and multiplex PCR amplification. Among them, there are currently two main methods of library construction with multiplex PCR products: adding adaptors to the product through a ligation reaction and adding adaptors through two rounds of PCR (the first round with primers having a universal sequence at the 5' end, and the second round with primers annealing to add adaptors). The library construction method including adding adaptors to multiplex PCR products is a mainstream library construction method.

The ligation effect of a TA sticky end is better than that of a blunt end, and now adding adaptors by TA ligation is mostly used. Some DNA polymerases can catalyze the addition of A bases at the 3' end of DNA fragments without relying on templates. This discovery laid the foundation for a series of technologies such as TA cloning and next-generation sequencing library construction. However, the current direct catalytic addition of A bases to the 3' end of DNA fragments is not efficient and is unstable.

### SUMMARY

Based on this, according to various embodiments of the present application, an amplification primer for sequencing library construction is provided, including a primer sequence fragment complementary to a target fragment and a base G ligated to the 5' end of the primer sequence fragment, wherein the amplification primer and the target fragment are not complementary at the base G.

In one embodiment, the base G is directly ligated to the 5' end of the primer sequence fragment.

In one embodiment, the base G is a base modified by phosphorylation.

According to various embodiments of the present application, use of the amplification primer described above in preparation of a kit for constructing a sequencing library is provided.

According to various embodiments of the present application, a method for constructing a sequencing library is provided, including:
providing a primer sequence fragment used for performing amplification and library construction on a deoxyribonucleic acid, the primer sequence fragment being complementary to a target fragment,
performing a treatment on the primer sequence fragment to obtain an amplification primer with a base G at the 5' end, wherein the treatment includes:
ligating a base G to the 5' end of the primer sequence fragment if the base at the 5' end of the primer sequence fragment is not G, or
ligating or not ligating a base G to the 5' end of the primer sequence fragment if the base at the 5' end of the primer sequence fragment is G;
performing cyclic amplification on the deoxyribonucleic acid using the amplification primer with a base G at the 5' end, to obtain an amplification fragment with a base C at the 3' end;
adding a base A to the 3' end of the amplification fragment; and
ligating an adaptor containing a T sticky end to the amplification fragment with base A added to the 3' end, wherein the adaptor contains a sequence required by a sequencing platform.

In one embodiment, the method further includes: phosphorylating the base G at the 5' end of the amplification primer before performing amplification on the deoxyribonucleic acid.

In one embodiment, the deoxyribonucleic acid is selected from the group consisting of a sample DNA, a sample plasmid, and a deoxyribonucleic acid obtained by reverse transcription of a sample RNA.

According to various embodiments of the present application, a sequencing library constructed by the above method for constructing a sequencing library is provided.

According to various embodiments of the present application, a kit for constructing a sequencing library is provided, including the amplification primer described above.

In one embodiment, the kit further includes at least one of a reagent for adding base A, a reagent for adding adaptors, a reagent for PCR amplification and a reagent for purification.

According to various embodiments of the present application, a sequencing method is provided, including: constructing a sequencing library for a sample using the above method for constructing a sequencing library, and performing sequencing.

In one embodiment, the sequencing method is used to perform on the sample any one of mutation site detection, mutation site detection, gene fusion detection, and pathogenic microorganism detection.

In one embodiment, the mutation site includes at least one of intestinal cancer mutation sites, cervical cancer mutation sites, and urothelial cancer mutation sites.

In one embodiment, the chromosome ploidy includes at least one of the chromosome ploidy of intestinal cancer, the chromosome ploidy of cervical cancer, and the chromosome ploidy of urothelial cancer.

In one embodiment, the gene fusion includes at least one of EML4-ALK gene fusion, CD74- ROSI gene fusion, CCDC6-RET gene fusion, NCOA4-RET gene fusion, and TPM3-NTRK1 gene fusion.

In one embodiment, the pathogenic microorganism includes at least one of influenza A virus, influenza B virus and SARS-CoV-2.

In one embodiment, the sequencing is high-throughput sequencing.

According to various embodiments of the present application, a mutation site detection kit is provided, including the amplification primer described above.

According to various embodiments of the present application, a chromosome ploidy detection kit is provided, including the amplification primer described above.

According to various embodiments of the present application, a gene fusion detection kit is provided, including the amplification primer described above.

According to various embodiments of the present application, a pathogenic microorganism detection kit is provided, including the amplification primer described above.

According to various embodiments of the present application, a TA cloning method is provided, including the steps of: performing PCR amplification using the amplification primer described above to add A to the end of an amplification product.

The details of one or more embodiments of the present application are set forth in the accompanying drawings and the description below. Other features, objects and advantages of the present application will become apparent from the description, the accompanying drawings, and the claims.

### BRIEF DESCRIPTION OF THE DRAWINGS

To better describe and illustrate the embodiments and/or examples of the application disclosed herein, reference may be made to one or more accompanying drawings. The additional details or examples used to describe the accompanying drawings are not to be construed as limiting the scope of any one of the disclosed applications, the presently described embodiments and/or examples, and the presently understood preferred mode of the application.
FIG. 1 is a schematic diagram of the amplicon library construction process for DNA using conventional primers and primers of the present application using high-fidelity multiple enzymes, according to an example of the present application.
FIG.2 is a schematic diagram of the amplicon library construction process for DNA using conventional primers and primers of the present application using non-high-fidelity multiple enzymes, according to an example of the present application.
FIG.3 is a schematic diagram of the amplicon library construction process for RNA using conventional primers and primers of the present application using high-fidelity multiple enzymes, according to an example of the present application.
FIG.4 is a schematic diagram of the amplicon library construction process for RNA using conventional primers and primers of the present application using non-high-fidelity multiple enzymes, according to an example of the present application.

### DETAILED DESCRIPTION OF THE EMBODIMENTS

In order to facilitate the understanding of the present application, the present application will be described more fully hereinafter with reference to the related accompanying drawings. Various embodiments of the present application are presented in the accompanying drawings. However, the present application may be embodied in many different forms and is not limited to the embodiments described herein. Rather, these embodiments are provided so that the understanding of the content of the present application will be more thorough.

All technical and scientific terms used herein have the same meaning as commonly understood by one of ordinary skill in the art to which the present application applies, unless otherwise defined. The terms used in the specification of the present application herein are for the purpose of describing specific embodiments only and are not intended to limit the present application.

The addition efficiency of 3' end dA varies depending on the template. For example, the addition efficiency of 3' end dA depends on the nucleotide base composition of the 3' end of DNA, and fluctuates between 4% and 75%. The corresponding A- addition efficiency in the case where the 3' end nucleotide of DNA is C, T, G, or A bases is 80-85%, 75-80%, 45-50%, or 20-30%, respectively.

Therefore, the efficiency of adding A to DNA fragments with different ends is different.

It is resolved in the present application that TA ligation efficiency is improved by adding G to the 5' end of the forward and reverse primers (it would not be needed to be added if the primer originally has a G at the 5' end) so that the 3' ends of the upper and lower complementary strands of the amplicon are both C, thus guaranteeing the addition of A to each chain with a equal probability and a maximized efficiency of 80-85% due to the 3' end being C, in a process of catalyzing the addition of A at the 3' end in a non-template-dependent manner.

Through the present application, a higher A-addition efficiency is ensured, and more templates can be added with adaptors through TA ligation, which improves the library conversion rate; and also reduces the processes such as terminal repairing, and reduces the decline of DNA recovery rate caused by complicated operations. This method is more conducive to the detection of low-frequency mutations.

Also, the cloning efficiency of this method is also better than that of conventional methods in the application of TA cloning of PCR products.

In first aspect, embodiments of the present application provide an amplification primer for sequencing library construction, including a primer sequence fragment complementary to a target fragment and a base G ligated to the 5' end of the primer sequence fragment, wherein the amplification primer and the target fragment are not complementary at the base G.

As used herein, the term "complementary" means that two nucleic acid sequences are capable of forming hydrogen bonds between each other according to the base pairing principle (Waston-Crick principle) and thereby forming a duplex. In the present application, the term "complementary" includes "substantially complementary" and "completely complementarity". As used herein, the term "completely complementarity" means that all bases in one nucleic acid sequence are capable of pairing with bases in the other nucleic acid strand without the presence of mismatches or gaps. As used herein, the term "substantially complementarity" means that a majority of the bases in one nucleic acid sequence is capable of pairing with bases in the other nucleic acid strand with mismatches or gaps (e.g., a mismatch or gap of one or more nucleotides) allowed to be present. Generally, under conditions that allow hybridization, annealing, or amplification of the nucleic acids, two nucleic acid sequences that are "complementary" (e.g., substantially complementary or completely complementary) will selectively/specifically hybridize or anneal to form a duplex.

As used herein, the terms "target fragment", "target amplification fragment" and "target sequence" refer to the target nucleic acid sequence to be amplified. In the present application, the terms "target fragment", "target amplification fragment" and "target sequence" have the same meaning and can be used interchangeably. It is easy to understand that the target fragment is specific for the sample sequence. In other words, under conditions that allow nucleic acid hybridization, annealing or amplification, the amplification primer only hybridizes or anneals to a specific target fragment to amplify the specific fragment, but does not hybridize or anneal to other nucleic acid sequences.

When the amplification primer is complementary to the target fragment and the primer sequence fragment is complementary to the target fragment, the amplification primer and the target tip are not complementary at base G.

In some embodiments, base G is directly ligated to the 5' end of the complementary sequence without other bases spaced in between.

In some embodiments, the base G is a base modified by phosphorylation. The 5' end of the primer is modified by phosphorylation to ensure the phosphorylation state of the 5' ends of the double-strand product. Phosphorylation of the 5' end of the primer can reduce the phosphorylation reaction and purification steps in library construction, thus saving time and reducing molecular loss caused by multiple operating steps. Adding G to the 5' end of the primer in combination with phosphorylation of the 5' end can improve the library conversion rate and reduce the loss rate.

In a second aspect, the embodiments of the present application provide the use of the above amplification primer in preparation of a kit for constructing a sequencing library, so as to ensure that the kit achieves a higher A-adding efficiency, so that more templates can be added with adaptors through TA ligation, which improves the library conversion rate; and the kit also reduces processes such as terminal repairing, and reduces the decline of DNA recovery rate caused by complicated operations.

In a third aspect, the embodiments of the present application provide a method for constructing a sequencing library, including:
providing a primer sequence fragment used for performing amplification and library construction on a deoxyribonucleic acid, the primer sequence fragment being complementary to a target fragment, and
performing a treatment on the primer sequence fragment to obtain an amplification primer with a base G at the 5' end, wherein the treatment includes:
ligating a base G to the 5' end of the primer sequence fragment if the base at the 5' end of the primer sequence fragment is not G, or
ligating or not ligating a base G to the 5' end of the primer sequence fragment if the base at the 5' end of the primer sequence fragment is G;
performing cyclic amplification on the deoxyribonucleic acid using the amplification primer, to obtain an amplification fragment with a base C at the 3' end;
adding a base A to the 3' end of the deoxyribonucleic acid; and
   ligating an adaptor containing a T sticky end to the amplification fragment with base A added to the 3' end, wherein the adaptor contains a sequence required by a sequencing platform.

On the basis that the addition efficiency of 3' end dA varies depending on the template, when the nucleotide base at the 3' end is a C base, the corresponding A-addition efficiency is the highest. In the above sequencing library construction method of the present application, base G is added to the 5' end (it would not be needed to be added additionally if G is originally present) of the PCR primer to ensure that the 3' end of the PCR product is C, ensuring that each product molecule has the same, higher A-addition efficiency. Since the efficiency of adding A to the ends of the product is improved, the ligation efficiency is improved in the reaction of adding adaptor in TA ligation. Compared with conventional library construction methods, the method of introducing modifications at the primer ends to obtain the amplification product which was conducive to the addition of A reduces the terminal repairing and other processes after amplification, and reduces decline of DNA recovery rate caused by complicated operations, has simple steps, saves reagents, and has higher ligation efficiency to adaptor. This method can also be used for PCR products in TA cloning, mutation site detection, chromosome ploidy detection, gene fusion detection, pathogenic microorganism detection, and other detection methods using sequencing.

The above deoxyribonucleic acid is selected from the group consisting of a sample DNA, a sample plasmid, and a deoxyribonucleic acid obtained by reverse transcription of a sample RNA.

In some embodiments, the method for constructing a sequencing library further includes the step of amplifying the ligation product using an amplification primer of the adaptor to achieve pre-amplification of the library.

In a fourth aspect, an embodiment of the present application provide a sequencing library constructed by the above method for constructing a sequencing library.

In a fifth aspect, an embodiment of the present application provide a kit for constructing a sequencing library.

A kit for constructing a sequencing library includes the amplification primers of any of the above embodiments.

The term "kit" refers to any article (e.g., package or container) that includes at least one device. The kit may further include any one of instructions for use, supplementary reagents, components, or assemblies, which are for use in the methods described herein or steps thereof.

Optionally, the kit also includes any one or more of a reagent for adding A, a reagent for adding adaptors, a reagent for PCR amplification and a reagent for purification.

Reagents for adding A include enzymes and dATP.

In some embodiments, the enzyme used to add A to 3' end is any one or more selected from the group consisting of klenowex-enzyme, Taq enzyme, and klenowex-enzyme with Taq enzyme. Alternatively, the addition of A to 3' end and ligation of the adaptor may be performed in one reaction system, or the addition of A to 3' end is performed followed by purification before ligation to the adaptor. Performing in one reaction system means that the sequencing adaptor is directly ligated without purification after adding A to the 3' end. Taq enzyme is preferred to be used for the addition of A to 3' end.

Reagents for adding adaptors include ligases and adaptors.

Ligase is any one or more selected from the group consisting of HiFi Taq DNA ligase, T4 RNA ligase 2, SplintR^{®} Ligase, 9°N^{™} DNA ligase, Taq DNA ligase, T7 DNA ligase, T3 DNA ligase, Electro Ligase, Blunt end/TA ligase premix, instant sticky ligase premix, T4 DNA ligase, Circligase ssDNA ligase, 5'AppDNA/RNA thermostable ligase.

The adaptor has a T base protruding at the 3' end, which is used for complementary pairing with the sample DNA fragment after adding A.

The reagents used for PCR amplification may be any one or more selected from the group consisting of DNA polymerases, dNTPs and DNA polymerase buffers.

Furthermore, the DNA polymerase is a combination of one or more enzymes selected from the group consisting of vent DNA polymerase, T7 DNA polymerase, Bsu DNA polymerase, T4 DNA polymerase, Klenow fragment, DNA polymerase I (E.coli), Therminator^{™}DNA polymerase, SulfolobusDNA polymerase IV, phi29 DNA Polymerase, Bst 2.0 DNA Polymerase, BstDNA Polymerase, Deep VentR^{®}DNA Polymerase, Deep VentR^{™}DNA Polymerase, VentR^{®}DNA Polymerase, EpiMark^{®} Hot Start Taq DNA Polymerase, LongAmp^{®} Hot Start Taq DNA Polymerase, LongAmp^{®} Taq DNA Polymerase, Taq DNA Polymerase Large Fragment, OneTaq^{®} Hot Start DNA Polymerase, Phusion^{®} Hot Start Flex DNA Polymerase, Phusion^{®} Ultra-Fidelity DNA Polymerase, and Q5^{®} Hot Start Q5^{®} ultra-fidelity DNA polymerase, etc.

Further, dNTPs may have a concentration of 2.5 mM, 10 mM, etc. DNA polymerase buffer is the best matching buffer selected for the selected polymerase.

The step of purifying DNA fragments may be performed by conventional methods in the art, for example, by using purification magnetic beads. Alternatively, reagents used for purification may include: 1.8× magnetic beads, 0.9× magnetic beads, and EB solution.

In the kit of the present application, the reagents are preferably packaged individually, but they can also be mixed-packaged on the premise of not affecting the implementation of the present application.

In a sixth aspect, a sequencing method is provided, including: constructing a sequencing library for a sample using the method for constructing a sequencing library of the above embodiments, and performing sequencing.

In the method of the present application, the sample may be any sample to be sequenced. For example, in certain embodiments, the sample contains or is DNA (e.g., genomic DNA or cDNA). In certain embodiments, the sample contains or is RNA (e.g., mRNA). In certain embodiments, the sample contains or is a mixture of nucleic acids (e.g., a mixture of DNA, a mixture of RNA, or a mixture of DNA and RNA).

In the methods of the present application, the sequence to be sequenced is not limited by its sequence composition or length. For example, the sequence to be sequenced may be DNA (e.g., genomic DNA or cDNA) or RNA molecules (e.g., mRNA). Furthermore, the target nucleic acid sequence to be detected may be single-stranded or doublestranded.

When the sample to be detected or the sequence to be sequenced is RNA, it is preferred to perform a reverse transcription reaction to obtain cDNA which is complementary to the mRNA before performing the method of the present application. For a detailed description of the reverse transcription reaction, see, for example, Joseph Sam-brook, et al., Molecular Cloning, A Laboratory Manual, Cold Spring Harbor Laboratory Press, Cold Spring Harbor, N. Y. (2001).

The sample to be sequenced or the sequence to be sequenced may be obtained from any sources, including but not limited to prokaryotes (such as bacteria), eukaryotes (such as protozoa, parasites, fungi, yeast, plants, animals including mammals and humans) or viruses (such as Herpes virus, HIV, influenza virus, Epstein-Barr virus, hepatitis virus, poliovirus, etc.) or viroids. The sample to be sequenced or the sequence to be sequenced may also be a nucleic acid sequence in any form, such as a genome sequence, an artificially isolated or fragmented sequence, a synthetic sequence, etc.

In some embodiments, the sample to be sequenced is blood, serum, plasma, cell culture supernatant, saliva, semen, tissues or tissue lysate.

In some embodiments of the present application, the sequencing method is high-throughput sequencing, also known as next-generation sequencing ("NGS"). Next-generation sequencing generates thousands to millions of sequences simultaneously in a parallel sequencing process. NGS is distinguished from "Sanger sequencing" (first-generation sequencing) in that the latter is based on the electrophoretic separation of chain termination products in a single sequencing reaction. Sequencing platforms for NGS that may be used in the present application may be commercially available, including but not limited to Illumina MiniSeq, NextSeq 550, life platform, etc.

This method may be used for non-diagnostic purposes, such as used for genetic research, and researches in racial distribution, human evolution and other fields (typically such as SNP applications).

In some embodiments, the sequencing methods of the above embodiments of the present application may be used for samples for mutation site detection, chromosome ploidy detection, gene fusion detection, pathogenic microorganism detection and other purposes.

In some embodiments, the mutation site includes at least one of an intestinal cancer mutation site, a cervical cancer mutation site, and an urothelial cancer mutation site.

In some embodiments, the chromosome ploidy includes at least one of the chromosome ploidy of intestinal cancer, the chromosome ploidy of cervical cancer, and the chromosome ploidy of urothelial cancer.

In some embodiments, the gene fusion includes at least one of EML4-ALK gene fusion, CD74- ROSI gene fusion, CCDC6-RET gene fusion, NCOA4-RET gene fusion, and TPM3-NTRK1 gene fusion.

In some embodiments, the pathogenic microorganism includes at least one of influenza A virus, influenza B virus and SARS-CoV-2.

In a seventh aspect, an embodiment of the present application provide a mutation site detection kit, including the amplification primer of any of the above embodiments.

In an eighth aspect, an embodiment of the present application provide a chromosome ploidy detection kit, including the amplification primer of any of the above embodiments.

In a ninth aspect, embodiments of the present application provide a gene fusion detection kit, including the amplification primer of any of the above embodiments.

In a tenth aspect, embodiments of the present application provide a pathogenic microorganism detection kit, including the amplification primer of any of the above embodiments.

In an eleventh aspect, a TA cloning method is provided, including the steps of: performing PCR amplification using the amplification primer of any of the above embodiments, thereby adding A to the terminal of an amplification product and improving the efficiency of adding A to the end of the PCR product.

### Example 1: Experimental procedures and methods

### (1) NGS related experiment

### 1. Library construction with multiplex PCR products using traditional and application methods.

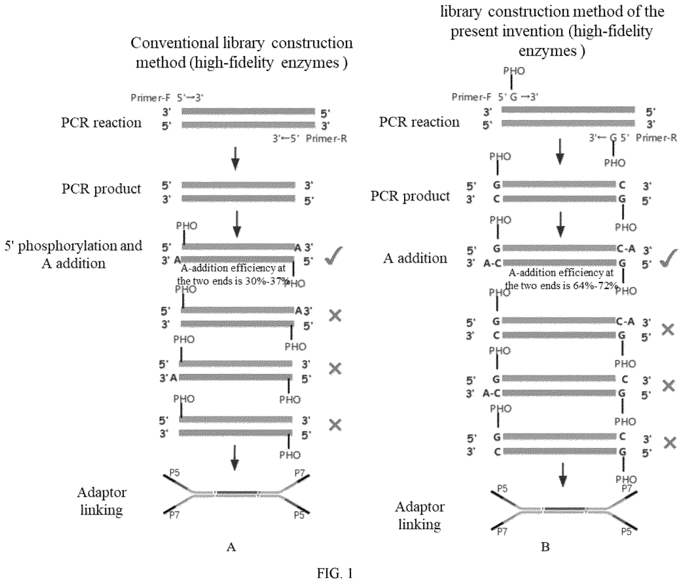

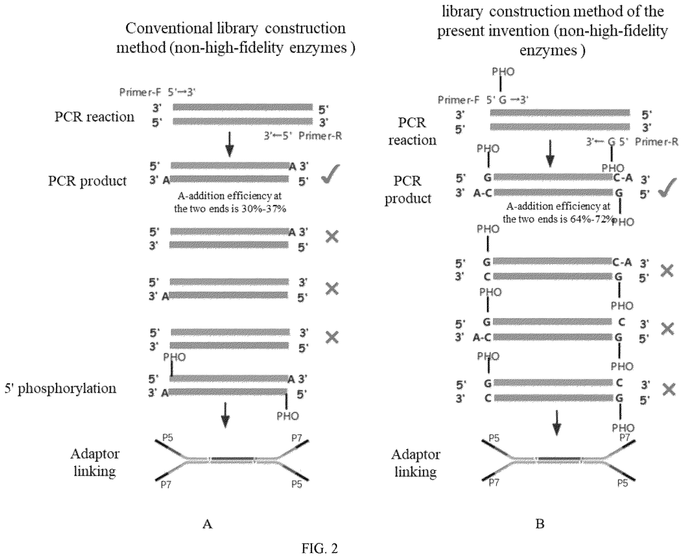

The library construction process for DNA is shown in FIGs. 1 and 2.

FIG.1: A. When constructing a library with a high-fidelity enzyme using a conventional method, the PCR product is subjected to 5' end phosphorylation and A-addition after PCR reaction, with A-addition efficiency at the two ends of only 30%-37%, and then an adaptor is ligated. B. When constructing a library with a high-fidelity enzyme using the method of the present application, A is directly added to the PCR product after PCR reaction, with A-addition efficiency at the two ends of 64%-72%, and then an adaptor is ligated.

FIG.2: A. When constructing a library with a non-high-fidelity enzyme using a conventional method, the PCR product is subjected to A-addition after the PCR reaction, with A-addition efficiency at the two ends of only 30%-37%, and then the 5' end is phosphorylated and the adaptor is ligated. B. When constructing a library with a non-high-fidelity enzyme using the method of the present application, after the PCR reaction, A has already been added to the 3' end of the PCR product and phosphorylation has been done at the 5' end, with A-addition efficiency at the two ends of 64%-72%, and the adaptor can be ligated directly.

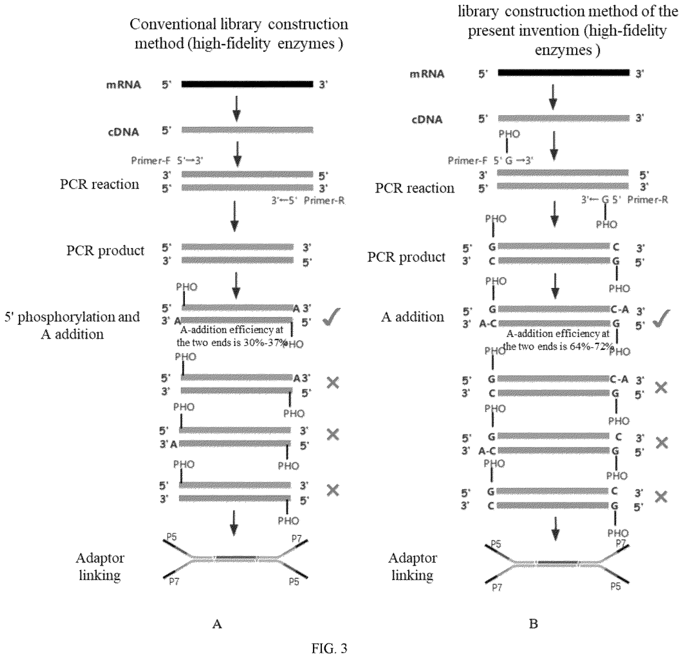

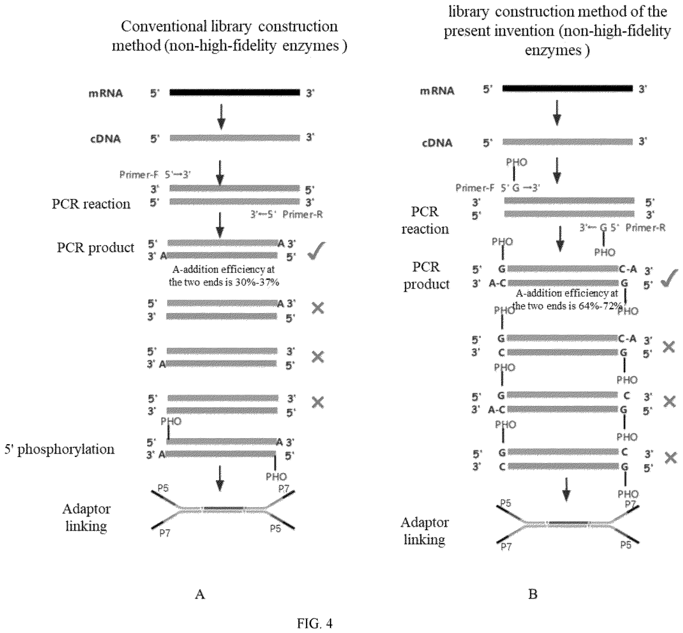

The RNA library construction process for RNA is shown in FIGs. 3 and 4.

FIG.3: A. When constructing a library with a high-fidelity enzyme using a conventional method, the mRNA is reverse transcribed into cDNA before performing PCR reaction, then the PCR product is subjected to 5' end phosphorylation and A-addition, with A-addition efficiency at the two ends of only 30%-37%, and then the adaptor is ligated; B. When constructing a library with a high-fidelity enzyme using the method of the present application, the mRNA is reverse transcribed into cDNA before performing PCR reaction, then A is directly added to the PCR product, with A-addition efficiency at the two ends of 64%-72%, and then the adaptor is ligated.

FIG.4: A. When constructing a library with a non-high-fidelity enzyme using a conventional method, the mRNA is reverse transcribed into cDNA before performing PCR reaction, and the PCR product is subjected to A-addition after PCR reaction, with A-addition efficiency at the two ends of only 30%-37%, and then the 5' end is phosphorylated and the adaptor is ligated.; B. When constructing a library with a high-fidelity enzyme using the method of the present application, the mRNA is reverse transcribed into cDNA before performing PCR reaction, and after the PCR reaction, A has already been added to the 3' end of the PCR product and phosphorylation has been done at the 5' end, with A-addition efficiency at the two ends of 64%-72%, and the adaptor can be ligated directly.

### 2. Detailed experiments

### 1) Sample collection:

Plasma samples: Whole blood was collected in EDTA blood collection tubes, and centrifuged at room temperature within 1 hour to obtain plasma. Centrifugation conditions: centrifuged at 1,500 g for 10 minutes. The supernatant was aspirated and collected, and then centrifuged again at 15,000 g for 10 minutes. The supernatant was aspirated and collected, which was the separated plasma.

Cervical exfoliated cell samples: Cervical exfoliated cell samples were collected in a ThinPrep cervical exfoliated cell preservation solution, stored at 4°C, and centrifuged at 12,000 rpm for 10 minutes. The supernatant was discarded, obtaining the cervical exfoliated cell pellet.

Urine samples: About 15-25 mL of morning urine was collected in a clean and dry disposable urine cup, and centrifuged at 500g for 10 minutes. The supernatant was discarded, obtaining the urothelial cell pellet.

Bowel cancer tissue samples: Fresh bowel cancer tissue, preserved in RNAlater.

### 2) Extraction and concentration determination of sample DNA:

Plasma DNA extraction: Plasma samples were subjected to extraction using a QIAAMP Circulating nucleic acid kit (QIAGEN-55114).

Extraction of gDNA from cervical exfoliated cells: CWBio Blood Genomic DNA Mini Kit (CW2087M) was used, and the starting volume of each sample was 2 mL.

DNA extraction from urine samples: CWBio Blood Genomic DNA Mini Kit (CW2087M) was used to extract DNA from urine samples.

Extraction of bowel cancer FFPE samples: PureLink^{™} Genomic DNA Mini Kit (Invitrogen) was used to extract DNA from bowel cancer tissue.

The concentration of DNA was determined by Qubit.

### 3) PCR targeted enrichment:

High-fidelity and non-high-fidelity multiplex PCR enzymes were used for PCR. The reaction system and conditions were in accordance with the instructions of the multiplex enzymes.

### 4) Subsequent reactions: For details of reactions such as A-addition, phosphorylation, and ligation, see the Examples.

### 5) Quality evaluation and concentration determination of the library:

The library concentration was determined by Qubit, and the reagent used was Qubit dsDNA HS Assay Kit, 500 assay (invitrogen/Q32854).

Library quality control: KAPA Library Quantification Kit Illumina^{®} Platforms (KK4824) was used to evaluate effective rate of the library.

### 6) Quality inspection of the library:

The resulting library was subjected to 2100 quality inspection using a high-sensitivity DNA kit (Agilent/5067-4626).

### 7) Sequencing on-machine for mixed samples:

Sequencing was performed using the next500 or novaseq of the Illumina platform.

### 8) Off-machine sequencing library analysis:

The test data were analyzed for mutation and ploidy using Jiangsu MicroDiag detection software and chromosome abnormality analysis software respectively, and the pathogenic chromosomes were analyzed using MicroDiag's in-house method.

### (2) TA cloning related experiments

Purg gene of zebrafish was PCR amplified, and the product was TA cloned. The TA cloning efficiency of PCR products with conventional primers and different modified primers were examined.

Zebrafish were purchased from Suzhou Murui Biotech Co., Ltd. For DNA extraction, firstly, the tail fins cut into pieces were homogenized using a tissue homogenizer, and then DNA was extracted using a DNeasy Blood & Tissue Kit (QIAGEN, Cat. No.: 69582), the concentration of DNA was measured by Qubit.

The kit used in the PCR reaction was Takara LA Taq DNA Polymerase (Takara Biotech), the amount of DNA template in the PCR reaction was 500ng, and amplification was carried out according to the PCR reaction system and conditions recommended in the instructions. QIAquick PCR Purification Kit (QIAGEN) was used for purification and recovery of PCR products. 2100 was used to check whether the size of the PCR fragment was correct and whether there were dimers. If there were dimers and non-specific amplification, gel cutting recovery should be performed with a GenElute^{™} gel recovery kit (Sigma-Aldrich). The purified product concentration was determined using Qubit.

The kit used in TA cloning was Mighty TA-cloning Kit (Takara Biotech). A T vector and the PCR product to be inserted were mixed at a molar ratio of 1:3 to ensure that the volume was 5 µL total. Then 5 µL of Ligation Mighty Mix was added and mixed well gently. The reaction was performed at 16°C for 30 minutes, and all added to 100 µl of competent cells (E. coli, DH5a) for transformation. Then the cells were spread on an L-agar plate containing X-Gal, IPTG, and Amp, and cultured at 37°C overnight. The Colony was directly subjected to PCR to confirm the recombinants.

### Example 2: Library effective rate

MRC-5 cell DNA was amplified using the four kinds of primers in Table 1 to verify the library effective rate of amplicon library construction using different primers.

The primer sequences and modifications are shown in Table 17, including 4 kinds of primers, ordinary primers (1), primer (2) that is the ordinary primers with phosphorylated 5' end, primer (3) that is is the ordinary primers with G added to 5' end, and primers (4) that is ordinary primers with G added to 5' end and phosphorylated, the concentration of each primer in each kind of primer pool was 400 nM. Multiplex PCRQIAGEN multiple enzyme was used to perform the PCR reaction in a system as follows. 10 µL of primer mixture was added, 30 ng of template was added, the other components were added following the instructions, and the system was finally made up to 50 µL with water. The reaction conditions were set according to the instructions. The product was purified using a PCR product purification kit (QIAGEN), and eluted in 20 µL. For reactions such as base A-addition, adaptor ligation, and library pre-amplification, see patent CN103298955B.

Qubit dsDNA HS Assay Kit, 500 assay (invitrogen/Q32854) and KAPA Library Quantification Kit Illumina^{®} Platforms (KK4824) were used to evaluate the effective rate of libraries constructed with different primers. The actual effective concentration was the library concentration measured using the KAPA Library Quantification Kit. Library effective rate = actual effective concentration/library concentration (Qubit). See Table 1 for details.

**Table 1 Library effective rate for library construction using four kinds of primers**

| Sample type | Ordinary primers | | | Primers with phosphorylated 5' end | | | Primers with G added to 5' end | | | Primers with G added to 5' end and phosphorylated | | |
|---|---|---|---|---|---|---|---|---|---|---|---|---|
| | Library conc. (Qubit) | Actual effectiv e conc. | Library effectiv e rate | Library conc. (Qubit) | Actual effective conc. | Library effective rate | Library conc. (Qubit) | Actual effective conc. | Library effective rate | Library conc. (Qubit) | Actual effective conc. | Library effective rate |
| HD734 | 2.16 | 1.38 | 63.89% | 2.12 | 1.79 | 84.43% | 2.23 | 1.99 | 89.24% | 2.65 | 2.53 | 95.47% |
| HD752 | 2.11 | 1.25 | 59.24% | 2.35 | 2.05 | 87.23% | 2.36 | 2.13 | 90.25% | 2.78 | 2.62 | 94.24% |
| HD813 | 2.26 | 1.61 | 71.24% | 2.26 | 2.01 | 88.94% | 2.51 | 2.33 | 92.83% | 2.91 | 2.78 | 95.53% |
| HD815 | 2.84 | 1.72 | 60.56% | 2.51 | 2.16 | 86.06% | 2.31 | 2.09 | 90.48% | 2.84 | 2.69 | 94.72% |
| Ref. A | 2.31 | 1.11 | 48.05% | 2.11 | 1.85 | 87.68% | 2.21 | 2.01 | 90.95% | 2.52 | 2.43 | 96.43% |
| Ref. B | 2.16 | 1.36 | 62.96% | 2.23 | 1.95 | 87.44% | 2.33 | 2.12 | 90.99% | 2.51 | 2.41 | 96.02% |
| Ref. C | 2.22 | 1.18 | 53.15% | 2.41 | 2.11 | 87.55% | 2.54 | 2.29 | 90.16% | 2.55 | 2.44 | 95.69% |
| Intestinal cancer sample | 1.97 | 1.12 | 56.85% | 1.91 | 1.61 | 84.29% | 2.31 | 2.04 | 88.31% | 2.63 | 2.49 | 94.68% |
| Healthy person plasma sample | 1.88 | 1.34 | 71.28% | 1.99 | 1.66 | 83.42% | 2.26 | 2.01 | 88.94% | 2.61 | 2.51 | 96.17% |
| Cervical exfoliated cell samples | 3.02 | 1.98 | 65.56% | 3.12 | 2.55 | 81.73% | 3.2 | 2.79 | 87.19% | 3.33 | 3.10 | 93.09% |
| urothelial | 2.88 | 2.11 | 73.26% | 3.01 | 2.39 | 79.40% | 3.12 | 2.64 | 84.62% | 3.29 | 2.99 | 90.88% |
| cancer sample | | | | | | | | | | | | |
| Healthy person's urine sample | 2.98 | 2.03 | 68.12% | 2.56 | 2.03 | 79.30% | 2.94 | 2.68 | 91.16% | 3.41 | 3.27 | 95.89% |

Result analysis: the library concentration and effective rate of the libraries constructed with the four kinds of primers were normal; the library effective rate for primers with G added to 5' end and phosphorylated was better than that of the primers with G added to 5' end; the library effective rate of the primers with G added to 5' end was better than that of the primers with phosphorylated 5' end; the library effective rate of the primers with phosphorylated 5' end is better than that of ordinary primers.

### Example 3: Detection of sites of commercial gDNA reference

Positive reference HD734 from Horizon and negative reference HD752 (100% Wildtype) were used to verify the detection effects of different primers for library construction.

The primer sequences and modifications are shown in Table 17, including 4 kinds of primers, ordinary primers (1), primer (2) that is the ordinary primers with phosphorylated 5' end, primer (3) that is is the ordinary primers with G added to 5' end, and primers (4) that is ordinary primers with G added to 5' end and phosphorylated, the concentration of each primer in each kind of primer pool was 400 nM. A kit (Phusion U multiplex PCR master mix, Thermo Scientific) was used to perform the multiplex PCR reaction in a PCR reaction system as follows. 10 µL of primer mixture was added, 30 ng of template was added, the other components were added following the instructions, and the system was finally made up to 50 µL with water. The reaction conditions were set according to the instructions. The product was purified using a PCR product purification kit (QIAGEN), and eluted in 20 µL. The product obtained using the first primers needed to be phosphorylated. T4 PNK enzyme from NEB was used, and the reaction system and conditions were set according to the instructions of the enzyme. The product was purified with the QIAGEN kit. See patent CN103298955B. The experiment was repeated 10 times. The detection results are shown in Table 2.

**Table 2 The detected numbers in the case of amplicon library construction using four kinds of primers**

| Reference | GENE | AA | AF | Detected number | Ordinary primers | Primers with phosphorylated 5' end | Primers with G added to 5' end | Primers with G added to 5' end and phosphorylated |
|---|---|---|---|---|---|---|---|---|
| HD734 | PIK3CA | H1047R | 30% | 10 | 10 | 10 | 10 | 10 |
| | KRAS | G13D | 25% | 10 | 10 | 10 | 10 | 10 |
| | BRAF | V600E | 8% | 10 | 10 | 10 | 10 | 10 |
| | KRAS | G12S | 1.3% | 10 | 6 | 7 | 9 | 10 |
| | PIK3CA | E542K | 1.3% | 10 | 6 | 8 | 8 | 9 |
| | BRAF | V600M | 1.0% | 10 | 5 | 6 | 8 | 10 |
| | EGFR | T790M | 1.0% | 10 | 4 | 5 | 8 | 10 |
| HD752 | FGFR2 | S252W | 0.00% | 10 | 0 | 0 | 0 | 0 |
| | FLT3 | D835Y | 0.00% | 10 | 0 | 0 | 0 | 0 |
| | GNA11 | Q209L | 0.00% | 10 | 0 | 0 | 0 | 0 |
| | GNAQ | Q209L | 0.00% | 10 | 0 | 0 | 0 | 0 |
| | IDH1 | R132H | 0.00% | 10 | 0 | 0 | 0 | 0 |
| | IDH2 | R140Q | 0.00% | 10 | 0 | 0 | 0 | 0 |
| | JAK2 | V617F | 0.00% | 10 | 0 | 0 | 0 | 0 |
| | KIT | D816V | 0.00% | 10 | 0 | 0 | 0 | 0 |

Results analysis: when testing the wild-type reference, none of the sites was detected using the four kinds of primers. For sites with higher mutation frequency (AF ≥ 8%), all the four kinds of primers were able to be detected in 10 times of detection; for low-frequency sites (1%≤MAF≤1.5%), for 4 low-frequency sites, the detection rate of the ordinary primers was 52.5%, the detection rate of the primers with phosphorylated 5' end was 65%, and the detection rate of primers with G added to 5' end was 82.5%, and the detection rate of primers with G added to 5' end and phosphorylated was 97.5%. It could be seen from the results that, compared to phosphorylation modification, adding G at the 5' end was more beneficial for low-frequency detection. If phosphorylation modification was performed in addition to adding G, the effect would be better.

### Example 4: Detection of sites of commercial cfDNA reference

Positive reference HD813 and negative reference HD815 (100% Wildtype) from Horizon were used to verify the detection effects of different primers for library construction.

The primer sequences and modifications are shown in Table 17, including 4 kinds of primers, ordinary primers (1), primer (2) that is the ordinary primers with phosphorylated 5' end, primer (3) that is is the ordinary primers with G added to 5' end, and primers (4) that is ordinary primers with G added to 5' end and phosphorylated, the concentration of each primer in each kind of primer pool was 400 nM. A kit (Phusion U multiplex PCR master mix, Thermo Scientific) was used to perform the multiplex PCR reaction in a PCR reaction system as follows. 10 µL of primer mixture was added, 30 ng of template was added, the other components were added following the instructions, and the system was finally made up to 50 µL with water. The reaction conditions were set according to the instructions. The product was purified using a PCR product purification kit (QIAGEN), and eluted in 20 µL. The product obtained using the first primers needed to be phosphorylated. T4 PNK enzyme from NEB was used, and the reaction system and conditions were set according to the instructions of the enzyme. The product was purified with the QIAGEN kit. See patent CN103298955B. The experiment was repeated 10 times. The detection results are shown in Table 3.

**Table 3 The detected numbers in the case of amplicon library construction using four kinds of primers**

| Reference | GENE | AA | AF | Detected number | Ordinary primers | Primers with phosphorylated 5' end | Primers with G added to 5' end | Primers with G added to 5' end and phosphorylated |
|---|---|---|---|---|---|---|---|---|
| HD813 | EGFR | L858R | 1.00% | 10 | 6 | 8 | 8 | 10 |
| | EGFR | ΔE746-A750 | 1.00% | 10 | 5 | 6 | 9 | 9 |
| | EGFR | T790M | 1.00% | 10 | 5 | 5 | 8 | 10 |
| | EGFR | V769-D770insASV | 1.00% | 10 | 7 | 7 | 7 | 10 |
| | KRAS | G12D | 1.30% | 10 | 6 | 7 | 9 | 10 |
| | NRAS | Q61K | 1.30% | 10 | 5 | 6 | 8 | 9 |
| | NRAS | A59T | 1.30% | 10 | 4 | 5 | 8 | 10 |
| | PIK3CA | E545K | 1.30% | 10 | 6 | 7 | 8 | 10 |
| HD815 | EGFR | L858R | 0.00% | 10 | 0 | 0 | 0 | 0 |
| | EGFR | ΔE746-A750 | 0.00% | 10 | 0 | 0 | 0 | 0 |
| | EGFR | T790M | 0.00% | 10 | 0 | 0 | 0 | 0 |
| | EGFR | V769-D770insASV | 0.00% | 10 | 0 | 0 | 0 | 0 |
| | KRAS | G12D | 0.00% | 10 | 0 | 0 | 0 | 0 |
| | NRAS | Q61K | 0.00% | 10 | 0 | 0 | 0 | 0 |
| | NRAS | A59T | 0.00% | 10 | 0 | 0 | 0 | 0 |
| | PIK3CA | E545K | 0.00% | 10 | 0 | 0 | 0 | 0 |

Results analysis: when testing the wild-type reference, none of the sites was detected using the four kinds of primers. For sites with 1%≤MAF≤1.5%, the detection rate of the ordinary primers was 53.8%, the detection rate of the primers with phosphorylated 5' end was 63.8%, and the detection rate of the primers with G added to 5' end was 81.3%, and the detection rate of the primers with G added to 5' end and phosphorylated was 97.5%. Comparing primers 1 and 2, it could be seen that phosphorylation of the 5' end is beneficial to the detection of low-frequency sites. Comparing primers 2 with primers 3, it could be seen that adding G to the 5' end is beneficial to the detection of low-frequency sites. By comprehensive comparison, adding G at the 5' end in combination with phosphorylation was more conducive to low-frequency mutation detection.

### Example 5: Detection of sites in self-made reference gDNA

Reference A containing multiple mutation sites was formulated by blending multiple cell lines. The mutation frequency of the sites is shown in Table 2. The four kinds of primers in Example 2 were used for detection. The multiplex PCR method was the same as in Example 2. The product obtained using the first primer was phosphorylated using Invitrogen's T4 PNK kit, and purified using the QIAGEN kit. For reactions such as A-addition, adaptor ligation, and library pre-amplification, see patent CN108251515A. The experiment was repeated 10 times. The detection results are shown in Table 4.

Results analysis: when testing the wild-type reference, none of the sites was detected using the four kinds of primers. For sites with 2%≤MAF≤5%, the detection rate of the ordinary primers was 85%, the detection rate of the primers with phosphorylated 5' end was 88.3%, and the detection rate of the primers with G added to 5' end was 90%, and the detection rate of the primers with G added to 5' end and phosphorylated was 100%. For sites with 0.5%≤MAF≤1%, the detection rate of the ordinary primers was 58.6%, the detection rate of the primers with phosphorylated 5' end was 71.4%, and the detection rate of the primers with G added to 5' end was 82.1%, and the detection rate of primer 4 was 93.6%. By comprehensive comparison, adding G at the 5' end in combination with phosphorylation was more conducive to low-frequency mutation detection.

**Table 4 The detected numbers in the case of amplicon library construction using four kinds of primers**

| GENE | AA | AF | Detected number | Ordinary primers | Primers with phosphorylated 5' end | Primers with G added to 5' end | Primers with G added to 5' end and phosphorylated |
|---|---|---|---|---|---|---|---|
| CTNNB1 | p.S45del | 2.68% | 10 | 9 | 9 | 9 | 10 |
| DNAH2 | p.T2172I | 0.63% | 10 | 6 | 8 | 9 | 10 |
| ERBB3 | p.N126K | 0.77% | 10 | 7 | 7 | 9 | 10 |
| FBXW7 | p.R465H | 0.97% | 10 | 6 | 7 | 8 | 9 |
| KRAS | p.G13D | 4.00% | 10 | 9 | 9 | 9 | 10 |
| PIK3CA | p.E545K | 0.98% | 10 | 5 | 6 | 8 | 9 |
| PIK3CA | p.D549N | 0.98% | 10 | 7 | 7 | 8 | 10 |
| PIK3CA | p.R88Q | 0.64% | 10 | 5 | 6 | 8 | 10 |
| PIK3CA | p.G118D | 0.96% | 10 | 8 | 8 | 9 | 9 |
| PPP2R1A | p.R183P | 0.66% | 10 | 5 | 6 | 8 | 10 |
| PTEN | p.R130X | 0.86% | 10 | 8 | 8 | 9 | 9 |
| PTEN | p.R233X | 0.99% | 10 | 8 | 7 | 8 | 9 |
| KRAS | G13D | 0% | 10 | 0 | 0 | 0 | 0 |
| BRAF | V600E | 0% | 10 | 0 | 0 | 0 | 0 |
| KRAS | G12S | 0% | 10 | 0 | 0 | 0 | 0 |
| EGFR | p.T790M | 5.00% | 10 | 9 | 9 | 10 | 10 |
| EGFR | p.T790M | 2.00% | 10 | 8 | 9 | 9 | 10 |
| EGFR | p.T790M | 1.00% | 10 | 6 | 8 | 9 | 10 |
| EGFR | p.T790M | 0.50% | 10 | 4 | 7 | 8 | 7 |
| PIK3CA | p.H1047R | 5.00% | 10 | 9 | 9 | 9 | 10 |
| PIK3CA | p.H1047R | 2.00% | 10 | 7 | 8 | 8 | 10 |
| PIK3CA | p.H1047R | 1.00% | 10 | 5 | 7 | 7 | 10 |
| PIK3CA | p.H1047R | 0.50% | 10 | 2 | 7 | 7 | 9 |

### Example 6: Detection of sites in self-made reference cfDNA

The extracted cell line DNA was fragmented to about 170 bp by ultrasound. After passing the 2100 quality inspection, reference A containing multiple mutation sites was formulated by blending multiple cell lines. The mutation frequency of each site is shown in Table 2. The four kinds of primers in Example 2 were used for detection. The multiplex PCR method was the same as in Example 2. The product obtained using the first primer was phosphorylated using Invitrogen's T4 PNK kit, and purified using the QIAGEN kit. For reactions such as A-addition, adaptor ligation, and library pre-amplification, see patent CN108251515A. The experiment was repeated 10 times. The detection results are shown in Table 5.

Results analysis: when testing the wild-type reference, none of the sites was detected using the four kinds of primers. For sites with 2%≤MAF≤5%, the detection rate of primer 1 was 52/60=86.7%, the detection rate of primer 2 was 56/60=93.3%, and the detection rate of primer 3 was 95%, and the detection rate of primer 4 was 98.33%. For sites with 0.5%≤MAF≤1%, the detection rate of primer 1 was 49.3%, the detection rate of primer 2 was 57.1%, and the detection rate of primer 3 was 70%, and the detection rate of primer 4 was 84.3%. By comprehensive comparison, primer 4 (with G added at the 5' end in combination with phosphorylation) is more conducive to low-frequency mutation detection.

**Table 5 The detected numbers in the case of amnlicon library construction using four kinds of nrimers**

| GENE | AA | AF | Detected number | Ordinary primers | Primers with phosphorylated 5' end | Primers with G added to 5' end | Primers with G added to 5' end and phosphorylated |
|---|---|---|---|---|---|---|---|
| CTNNB1 | p.S45del | 2.68% | 10 | 9 | 10 | 10 | 10 |
| DNAH2 | p.T2172I | 0.63% | 10 | 4 | 5 | 6 | 9 |
| ERBB3 | p.N126K | 0.77% | 10 | 4 | 5 | 6 | 8 |
| FBXW7 | p.R465H | 0.97% | 10 | 5 | 6 | 7 | 9 |
| KRAS | p.G13D | 4.00% | 10 | 10 | 10 | 10 | 10 |
| PIK3CA | p.E545K | 0.98% | 10 | 5 | 7 | 8 | 9 |
| PIK3CA | p.D549N | 0.98% | 10 | 4 | 7 | 8 | 9 |
| PIK3CA | p.R88Q | 0.64% | 10 | 4 | 7 | 8 | 9 |
| PIK3CA | p.G118D | 0.96% | 10 | 5 | 6 | 6 | 7 |
| PPP2R1A | p.R183P | 0.66% | 10 | 6 | 7 | 8 | 10 |
| PTEN | p.R130X | 0.86% | 10 | 5 | 8 | 8 | 9 |
| PTEN | p.R233X | 0.99% | 10 | 6 | 8 | 8 | 8 |
| KRAS | G13D | 0% | 10 | 0 | 0 | 0 | 0 |
| BRAF | V600E | 0% | 10 | 0 | 0 | 0 | 0 |
| KRAS | G12S | 0% | 10 | 0 | 0 | 0 | 0 |
| EGFR | p.T790M | 5.00% | 10 | 8 | 9 | 9 | 10 |
| EGFR | p.T790M | 2.00% | 10 | 6 | 8 | 8 | 9 |
| EGFR | p.T790M | 1.00% | 10 | 5 | 5 | 7 | 7 |
| EGFR | p.T790M | 0.50% | 10 | 2 | 3 | 5 | 7 |
| PIK3CA | p.H1047R | 5.00% | 10 | 10 | 10 | 10 | 10 |
| PIK3CA | p.H1047R | 2.00% | 10 | 9 | 9 | 10 | 10 |
| PIK3CA | p.H1047R | 1.00% | 10 | 6 | 7 | 8 | 9 |
| PIK3CA | p.H1047R | 0.50% | 10 | 3 | 4 | 5 | 8 |

### Example 7: Detection of sites in nucleosomes

Nucleosomes were prepared using EpiScope^{®} Nucleosome Preparation Kit (Takara Code No. 5333). Reference C containing multiple mutation sites was formulated by blending the nucleosomes prepared from multiple cell lines. The mutation frequency of each site is shown in Table 2. The four kinds of primers in Example 2 were used for detection. The multiplex PCR method was the same as in Example 2. The product obtained using the first primer was phosphorylated using Invitrogen's T4 PNK kit, and purified using the QIAGEN kit. For reactions such as A-addition, adaptor ligation, and library pre-amplification, see patent CN108251515A. The experiment was repeated 10 times. The detection results are shown in Table 6.

Results analysis: when testing the wild-type reference, none of the sites was detected using the four kinds of primers. For sites with 2%≤MAF≤5%, the detection rate of primer 1 was 86.7%, the detection rate of primer 2 was 93.3%, and the detection rate of primer 3 was 95%, and the detection rate of primer 4 was 98.3%. For sites with 0.5%≤MAF≤1%, the detection rate of primer 1 was 50.7%, the detection rate of primer 2 was 62.1%, and the detection rate of primer 3 was 75%, and the detection rate of primer 3 was 86.4%. By comprehensive comparison, primer 4 (with G added at the 5' end in combination with phosphorylation) is more conducive to low-frequency mutation detection.

**Table 6 The detected numbers in the case of amplicon library construction using four kinds of primers**

| GENE | AA | AF | Detected number | Ordinary primer | Primers with phosphorylated 5' end | Primers with G added to 5' end | Primers with G added to 5' end and phosphorylated |
|---|---|---|---|---|---|---|---|
| CTNNB1 | p.S45del | 2.68% | 10 | 9 | 10 | 10 | 10 |
| DNAH2 | p.T2172I | 0.63% | 10 | 4 | 5 | 7 | 8 |
| ERBB3 | p.N126K | 0.77% | 10 | 4 | 5 | 7 | 9 |
| FBXW7 | p.R465H | 0.97% | 10 | 5 | 6 | 7 | 9 |
| KRAS | p.G13D | 4.00% | 10 | 10 | 10 | 10 | 10 |
| PIK3CA | p.E545K | 0.98% | 10 | 6 | 8 | 8 | 10 |
| PIK3CA | p.D549N | 0.98% | 10 | 6 | 7 | 8 | 9 |
| PIK3CA | p.R88Q | 0.64% | 10 | 6 | 7 | 8 | 9 |
| PIK3CA | p.G118D | 0.96% | 10 | 5 | 6 | 9 | 8 |
| PPP2R1A | p.R183P | 0.66% | 10 | 6 | 7 | 8 | 10 |
| PTEN | p.R130X | 0.86% | 10 | 5 | 8 | 8 | 9 |
| PTEN | p.R233X | 0.99% | 10 | 7 | 8 | 8 | 8 |
| KRAS | G13D | 0% | 10 | 0 | 0 | 0 | 0 |
| BRAF | V600E | 0% | 10 | 0 | 0 | 0 | 0 |
| KRAS | G12S | 0% | 10 | 0 | 0 | 0 | 0 |
| EGFR | p.T790M | 5.00% | 10 | 7 | 9 | 9 | 10 |
| EGFR | p.T790M | 2.00% | 10 | 6 | 8 | 8 | 10 |
| EGFR | p.T790M | 1.00% | 10 | 5 | 5 | 7 | 7 |
| EGFR | p.T790M | 0.50% | 10 | 3 | 4 | 5 | 8 |
| PIK3CA | p.H1047R | 5.00% | 10 | 10 | 10 | 10 | 10 |
| PIK3CA | p.H1047R | 2.00% | 10 | 9 | 9 | 10 | 10 |
| PIK3CA | p.H1047R | 1.00% | 10 | 6 | 7 | 9 | 9 |
| PIK3CA | p.H1047R | 0.50% | 10 | 3 | 4 | 7 | 8 |

### Example 8: Detection of sites in intestinal cancer plasma samples

21 plasma samples from intestinal cancer and 10 plasma samples from healthy humans were collected. DNA was extracted and subjected to multiplex PCR using the four kinds of primers in Table 17. The multiplex PCR amplification was performed using Vazyme multiplex enzymes, and the PCR products were subjected to experiments such as A-addition, adaptor ligation, and library pre-amplification using the KAPA library construction kit. The detection results are shown in Table 7.

**Table 7 Detection of sites in intestinal cancer samples**

| Sample type | Samp le No. | Ordinary primer | Primers with phosphorylated 5' end | Primers with G added to 5' end | Primers with G added to 5' end and phosphorylated |
|---|---|---|---|---|---|
| Intestinal cancer | 1 | / | / | KRAS p.G13D 0.3% | KRAS p.G13D 0.3% |
| Intestinal cancer | 2 | / | / | / | / |
| Intestinal cancer | 3 | KRAS p.Q61H 0.3% | KRAS p.Q61H 0.4% | KRAS p.Q61H 1.1% | KRAS p.Q61H 1.4% |
| Intestinal cancer | 4 | KRAS p.G12D 0.5% | KRAS p.G12D 0.5% | KRAS p.G12D 1.7% | KRAS p.G12D 1.9% |
| Intestinal cancer | 5 | KRAS p.G13D 0.4% | KRAS p.G13D 0.6% | KRAS p.G13D 0.9% | KRAS p.G13D 1.2% |
| Intestinal cancer | 6 | / | / | BRAF p.V600E 0.4% | BRAF p.V600E 0.5% |
| | | | | | TP53 p.R273C 0.3% |
| Intestinal cancer | 7 | KRAS p.G12D 0.2% | KRAS p.G12D 0.5% | KRAS p.G12D 1.2% | KRAS p.G12D 1.3% |
| Intestinal cancer | 8 | / | / | KRAS p.G12C 0.3% | KRAS p.G12C 0.3% |
| Intestinal cancer | 9 | / | / | / | / |
| Intestinal cancer | 10 | BRAF p.V600E 0.7% | BRAF p.V600E 0.8% | BRAF p.V600E 2.1% | BRAF p.V600E 2.5% |
| | | | | | TP53 p.R273C 0.4% |
| Intestinal cancer | 11 | / | / | / | / |
| Intestinal cancer | 12 | / | / | KRAS p.G12V 0.3% | KRAS p.G12V 0.4% |
| | | | | APC p.R1450* 0.2% | APC p.R1450* 0.2% |
| Intestinal cancer | 13 | BRAF p.G12D 0.3% | BRAF p.G12D 0.4% | BRAF p.G12D 1.1% | BRAFp.G12D 1.3% |
| | | | | | TP53 p.R282W 0.3% |
| Intestinal cancer | 14 | / | / | KRAS p.G12S 0.3% | KRAS p.G12S 0.4% |
| Intestinal cancer | 15 | / | KRAS p.G12C 0.4% | KRAS p.G12C 1% | KRAS p.G12C 1.2% |
| Intestinal cancer | 16 | KRAS p.G12R 0.9% | KRAS p.G12R 1.2% | KRAS p.G12R 1.8% | KRAS p.G12R 2% |
| | | | | TP53 p.R273H 0.3% | TP53 p.R273H 0.5% |
| Intestinal cancer | 17 | / | / | / | / |
| Intestinal cancer | 18 | / | / | / | / |
| Intestinal cancer | 19 | / | / | / | BRAF p.V600E 0.4% |
| Intestinal cancer | 20 | / | / | KRAS p.G12D 0.4% | KRAS p.G12D 0.4% |
| | | | | | APC p.R876* 0.3% |
| Intestinal cancer | 21 | KRAS p.G12V 0.6% | KRAS p.G12V 0.8% | KRAS p.G12V 1.7% | KRAS p.G12V 1.8% |
| Healthy person | 22 | / | / | | / |
| Healthy person | 23 | / | / | | / |
| Healthy person | 24 | / | / | | / |
| Healthy person | 25 | / | / | | / |
| Healthy person | 26 | / | / | | / |
| Healthy person | 27 | / | / | | / |
| Healthy person | 28 | / | / | | / |
| Healthy person | 29 | / | / | | / |
| Healthy person | 30 | / | / | | / |
| Healthy person | 31 | / | / | | / |

All healthy humans presented negative results in the detection using the 4 kinds of primers. For the CRC detection, it was detected in 8 samples in the case where amplicon library construction was performed using the ordinary primers (single-site detection for all) with a detection rate of 38.1%, in 9 samples when using the primers with phosphorylated 5' end with the detection rate of 42.9% (single-site detection for all), in 15 samples using the primers with G added to 5' end with a detection rate of 71.4% (double-site detection for 2 samples), and in 16 samples when using the primers with G added to 5' end and phosphorylated with a detection rate of 76.2% (double-site detection for 6 samples). It could be seen that adding G to the 5' end of the primer significantly improved the detection rate. If G was added in combination with phosphorylation, the detection would be more stable.

### Table 9 Detection of sites in cervical exfoliated cell samples

Cervical exfoliated cell samples with clear pathological information were collected, including 15 cases of cervical cancer, 5 cases of CIN3, 5 cases of CIN2, and 5 cases of CIN1, and then multiplex PCR was performed on these 30 samples using the 4 kinds of primers in Table 17. The multiplex PCR amplification was performed using Vazyme multiplex enzymes, and the PCR products were subjected to experiments such as A-addition, adaptor ligation, and library pre-amplification using the NEB library construction kit. The detection results are shown in Table 8.

**Table 8 Detection of sites in cervical exfoliated cell samples**

| Sample type | Sample No. | Ordinary primer | Primers with phosphorylated 5' end | Primers with G added to 5' end | Primers with G added to 5' end and phosphorylated |
|---|---|---|---|---|---|
| Cervical cancer | 1 | PIK3CA p.E545K 17.2% | PIK3CAp.E545K 18.9% | PIK3CAp.E545K 17.4% | PIK3CAp.E545K 16.3% |
| | | | PIK3CAp.E726K 1.3% | | PIK3CAp.E453K 1.4% |
| | | | | PIK3CAp.E453K 1.4% | FBXW7 p.R479G 1.3% |
| Cervical cancer | 2 | / | / | PIK3CA p.E726K 1.3% | PIK3CAp.E726K 1.7% |
| Cervical cancer | 3 | PIK3CA p.E545K 16..3% | PIK3CA p.E545K 17.3% | PIK3CA p.E545K 17.4% | PIK3CA p.E545K 17.4% |
| Cervical cancer | 4 | ERBB2 p.S310Y 21.5% | ERBB2 p.S310Y 22.5% | ERBB2 p.S310Y 21.2% | ERBB2 p.S310Y 22.6% |
| Cervical cancer | 5 | PIK3CA p.E545K 18.9% | PIK3CA p.E545K 20.6% | PIK3CA p.E545K 20.1% | PIK3CA p.E453K 1.5% |
| | | | | | PIK3CA p.E545K 20.4% |
| | | | FBXW7 p.R479G 2.7% | FBXW7 p.R479G 1.3% | FBXW7 p.R479G 1.6% |
| Cervical cancer | 6 | / | / | PIK3CA p.E726K 1.6% | PIK3CA p.E726K 1.6% |
| Cervical cancer | 7 | PIK3CA p.H1047R 17.2% | PIK3CA p.H1047R 18.5% | PIK3CA p.H1047R 18.1% | PIK3CA p.H1047R 19.1% |
| Cervical cancer | 8 | PIK3CA p.E542K 51% | PIK3CA p.E542K 53% | PIK3CA p.E542K 50.6% | PIK3CA p.E542K 52.6% |
| Cervical cancer | 9 | / | PIK3CA p.K111del 4.8% | PIK3CA p.K111del 3.2% | PIK3CA p.K111del 3.2% |
| | | | PTEN p.G165E 2.2% | PTEN p.G165E 2.2% | PTEN p.G165E 2.2% |
| Cervical cancer | 10 | PIK3CA p.E726K 2.2% | PIK3CA p.E726K 2.5% | PIK3CA p.E726K 2.5% | PIK3CA p.E726K 2.5% |
| Cervical cancer | 11 | CDKN2A p.W110X 16.9% | CDKN2A p.W110X 18.9% | CDKN2A p.W110X 20.9% | CDKN2A p.W110X 20.9% |
| Cervical cancer | 12 | / | / | PIK3CA p.E542K 1.9% | PIK3CA p.E542K 1.9% |
| Cervical cancer | 13 | / | / | | PIK3CA p.E545K 1.4% |
| Cervical cancer | 14 | / | | FBXW7 p.R479G 2.7% | FBXW7 p.R479G 2.7% |
| Cervical cancer | 15 | PIK3CA p.E545K 6.5% | PIK3CA p.E545K 7.2% | PIK3CA p.E545K 8.8% | PIK3CA p.E545K 8.6% |
| | | | PIK3CA p.E726K 1.2% | PIK3CA p.E726K 1.3% | PIK3CA p.E726K 1.5% |
| CIN3 | 16 | / | / | PIK3CA p.N107S 1.5% | PIK3CA p.N107S 1.2% |
| CIN3 | 17 | / | PIK3CA p.E542K 6.5%; | PIK3CA p.E542K 6.8%; | PIK3CA p.E542K 6.6%; |
| | | | | PIK3CA p.E545K 8.4%; | PIK3CA p.E545K 9.4%; |
| | | | PIK3CA p.E545K 9.9%; | PIK3CA p.N107S 1.3% | PIK3CA p.N107S 1.2% |
| CIN3 | 18 | / | / | / | PIK3CA p.E545K 1.4%; |
| CIN3 | 19 | / | PIK3CA p.E542K 7.9% | PIK3CA p.E542K 7.6% | PIK3CA p.E542K 7.4% |
| | | | PIK3CA p.E545K 9% | PIK3CA p.E545K 8.9% | PIK3CA p.E545K 8.8% |
| CIN3 | 20 | / | / | / | / |
| CIN2 | 21 | / | PIK3CA p.G118D 12.1% | PIK3CA p.G118D 11.8% | PIK3CA p.G118D 11.1% |
| CIN2 | 22 | / | / | / | / |
| CIN2 | 23 | / | / | / | / |
| CIN2 | 24 | / | / | PIK3CA p.E542K 1.8% | PIK3CA p.E542K 1.4% |
| CIN2 | 25 | / | / | / | PIK3CA p.E542K 1.4% |
| CIN1 | 26 | / | / | / | / |
| CIN1 | 27 | / | / | / | / |
| CIN1 | 28 | / | / | / | / |
| CIN1 | 29 | / | / | / | / |
| CIN1 | 30 | / | / | / | / |

In the library of cervical cancer samples, the detection rate of primer 1 was 9/15=60%, and the detection rate of cervical cancer in the case where the library was constructed with primer 2 was 10/15=66.7%. The detection rate of primer 3 was 13/15=86.7%, and the detection rate of cervical cancer in the case where the library was constructed with primer 4 was 15/15=100%. In CIN3, the detection rate of primer 1 was 0/5=0%, the detection rate of primer 2 was 2/5=40%; the detection rate of primer 3 was 3/5=60%, and the detection rate of primer 4 was 4/5=80%. In CIN2, the detection rate of primer 1 was 0/5=0%, the detection rate of primer 2 was 1/5=20%; the detection rate in the library constructed with primer 3 was 2/5=40%, and the detection rate of primer 4 was 3/5=60%. No sites were detected in the CIN1 sample using the library constructed with four kinds of primers. Primer 4 (with G added at the 5' end in combination with phosphorylation) could detect more chromosomal abnormalities.

### Example 10: Detection of sites in urine samples

20 urine samples from urothelial cancer and 10 urine samples from healthy humans were collected. DNA was extracted and subjected to multiplex PCR using the four kinds of primers. Thermo Fisher reagents were used in multiplex PCR, and the PCR products were subjected to experiments such as A-addition, adaptor ligation, and library amplification using the Vazyme library construction kit. The detection results are shown in Table 9.

**Table 9 Detection of sites in urine samples**

| Sample type | Sample No. | Ordinary primer | Primers with phosphorylated 5' end | Primers with G added to 5' end | Primers with G added to 5' end and phosphorylated |
|---|---|---|---|---|---|
| Urothelial cancer | 1 | KRAS c.735G>T 3.1% | TERT c.54C>A 2.5% | TERT c.54C>A 2.1% | TERT c.54C>A 2.3% |
| | | | KRAS c.735G>T 3.4% | KRAS c.735G>T 3.9% | KRAS c.735G>T 4.1% |
| Urothelial cancer | 2 | / | FGFR3 c.742C>T 3.2% | FGFR3 c.742C>T 3.5% | FGFR3 c.742C>T 3.5% |
| | | | | | HRAS c.182A>T 1.2% |
| | | | KRAS c.57G>C 4.1% | KRAS c.57G>C 4.3% | KRAS c.57G>C 4.3% |
| Urothelial cancer | 3 | / | / | / | / |
| Urothelial cancer | 4 | KRAS c.76A>T 4.1% | KRAS c.76A>T 4.5% PIK3CA c.278G>A 1.4% | KRAS c.76A>T 4.5% PIK3CA c.278G>A 1.4% | KRAS c.76A>T 4.1% ERBB2 c.308G>A 1.3% PIK3CA c.278G>A 1.5% |
| Urothelial cancer | 5 | / | / | / | FGFR3 c.1111A>T 1.5% |
| Urothelial cancer | 6 | ERBB2 c.914C>G 4.2% | ERBB2 c.914C>G 4.1% | ERBB2 c.914C>G 4.1% | ERBB2 c.914C>G 3.9% |
| Urothelial cancer | 7 | / | / | / | HRAS c.38G>A 0.98% |
| Urothelial cancer | 8 | / | | TERT c.93T>G 1.9% | TERT c.93T>G 2.3% |
| | | | | TERT c.124C>A 2.5% | TERT c.124C>A 2.3% |
| Urothelial cancer | 9 | | KRAS c.38G>A 3.1% | KRAS c.38G>A 3.5% | KRAS c.38G>A 4.1% |
| | | | TERT c.80C>T 2.1% | TERT c.80C>T 2.5% | TERT c.80C>T 2.3% |
| Urothelial cancer | 10 | FGFR3 c.746C>G 3.6% | FGFR3 c.746C>G 3.6% | FGFR3 c.746C>G 3.2% | FGFR3 c.746C>G 3.5% |
| | | ERBB2 c.291G>C 3.2% | ERBB2 c.291G>C 3.4% | ERBB2 c.291G>C 3.1% | ERBB2 c.291G>C 3.9% |
| Urothelial cancer | 11 | / | / | HRAS c.19G>C 3.3% | HRAS c.19G>C 5.2% |
| Urothelial cancer | 12 | PIK3CA c.317G>T 5.8% | PIK3CA c.317G>T 5.8% | PIK3CA c.317G>T 5.4% | PIK3CA c.317G>T 6.4% |
| Urothelial cancer | 13 | / | / | / | HRAS c.37G>C 1.2% |
| | | | | | ERBB2 c.829G>T 1.9% |
| Urothelial cancer | 14 | PIK3CA c.316G>C 5.2% | PIK3CA c.316G>C 5.2% | PIK3CA c.316G>C 5.1% | PIK3CA c.316G>C 5.4% |
| | | | | | FGFR3 c.1102G>T 1.5% |
| Urothelial cancer | 15 | PIK3CA c.1357G>A 5.5% | PIK3CA c.1357G>A 5.8% | PIK3CA c.1357G>A 5.4% | PIK3CA c.1357G>A 6.1% |
| | | ERBB2 c.1979G>A 2.9% | ERBB2 c.1979G>A 2.1% | ERBB2 c.1979G>A 2.3% | ERBB2 c.1979G>A 3.1% |
| Urothelial cancer | 16 | / | / | / | / |
| Urothelial cancer | 17 | / | / | PIK3CA c.323G>A 4.5% | PIK3CA c.323G>A 4.7% |
| | | | | HRAS c.218G>A 1.6% | HRAS c.218G>A 1.2% |
| Urothelial cancer | 18 | / | / | / | / |
| Urothelial cancer | 19 | / | / | / | / |
| Urothelial cancer | 20 | / | HRAS c.34G>A 3.5% | HRAS c.34G>A 3.2% | HRAS c.34G>A 3.5% |
| | | | | FGFR3 c.749C>A 0.5% | FGFR3 c.749C>A 1.3% |
| Healthy person | 21 | / | / | / | / |
| Healthy person | 22 | / | / | / | / |
| Healthy person | 23 | / | / | / | / |
| Healthy person | 24 | / | / | / | / |
| Healthy person | 25 | / | / | / | / |
| Healthy person | 26 | / | / | / | / |
| Healthy person | 27 | / | / | / | / |
| Healthy person | 28 | / | / | / | / |
| Healthy person | 29 | / | / | / | / |
| Healthy person | 30 | / | / | / | / |

All healthy humans presented negative results in the detection. For the detection in urothelial cancer samples, the detection rate in the case where amplicon library construction was performed using the ordinary primer was 7/20=35%, and the detection rate in the case where amplicon library construction was performed using the primers with phosphorylated 5' end was 10/20= 50%, the detection rate in the case where amplicon library construction was performed using the primers with G added to 5' end was 13/20=65%, the detection rate in the case where amplicon library construction was performed using the primers with G added to 5' end and phosphorylated was 16/20=80%, showing the best detection effect.

### Example 11: Ploidy detection limit

The positive cell line reference hela cells with 6 copies of chromosome 5 verified by FISH were blended with the negative cell line with 2 copies in different proportions to obtain references with different copy numbers. See Table 10 for details. The four kinds of primers in Table 18 were used for amplification respectively, and multiplex high-fidelity enzyme from Thermo Fisher was used for amplification. For the experiments of A-addition, adaptor ligation, and library pre-amplification of the amplicons, see patent CN103298955B. The detection results are shown in Table 10.

**Table 10 Ploidy detection limit**

| | Blending ratio | Theoretical copy number | Duplicate 1 | | | Duplicate 2 | | |
|---|---|---|---|---|---|---|---|---|
| | | | GAIN | LOSS | AI | GAIN | LOSS | AI |
| Ordinary primer | PC | 6 | √ | × | × | √ | × | × |
| | NC | 2 | × | × | × | × | × | × |
| | 50% | 4 | √ | × | × | √ | × | × |
| | 20% | 2.8 | √ | × | × | √ | × | × |
| | 10% | 2.4 | × | × | √ | × | × | × |
| | 5% | 2.2 | × | × | × | × | × | × |
| | 1% | 2.04 | × | × | × | × | × | × |
| Primers with phosphorylated 5' end | PC | 6 | √ | × | × | √ | × | × |
| | NC | 2 | × | × | × | × | × | × |
| | 50% | 4 | √ | × | × | √ | × | × |
| | 20% | 2.8 | √ | × | × | √ | × | × |
| | 10% | 2.4 | × | × | √ | × | × | √ |
| | 5% | 2.2 | × | × | √ | × | × | × |
| | 1% | 2.04 | × | × | × | × | × | × |
| Primers with G added to 5' end | PC | 6 | √ | × | × | √ | × | × |
| | NC | 2 | × | × | × | × | × | × |
| | 50% | 4 | √ | × | × | √ | × | × |
| | 20% | 2.8 | √ | × | × | √ | × | × |
| | 10% | 2.4 | × | × | √ | × | × | √ |
| | 5% | 2.2 | × | × | √ | × | × | √ |
| | 1% | 2.04 | × | × | × | × | × | × |
| Primers with G added to 5' end and phosphorylated | PC | 6 | √ | × | × | √ | × | × |
| | NC | 2 | × | × | × | × | × | × |
| | 50% | 4 | √ | × | × | √ | × | × |
| | 20% | 2.8 | √ | × | × | √ | × | × |
| | 10% | 2.4 | √ | × | × | √ | × | × |
| | 5% | 2.2 | √ | × | × | √ | × | × |
| | 1% | 2.04 | × | × | √ | × | × | √ |

The results of tests in duplicate showed that: for amplification with the ordinary primers, ploidy could be stably detected with the lowest detection limit of of 2.8 copies; the lowest detection limit for the primers with phosphorylated 5' end was 2.4 copies; for the amplification with the primers with G added to 5' end, the lowest detection limit was 2.2 copies; for amplification with the primers with G added to 5' end and phosphorylated, the lowest detection limit was 2.04 copies, showing the best detection effect (√ indicated that chromosome 5 was detected as positive, × indicated that chromosome 5 was not detected).

### Example 12: Detection of ploidy in intestinal cancer FFPE samples

20 FFPE samples of intestinal cancer with clear pathological information were collected. Amplification was performed using the four kinds of primers in Table 18. KAPA high-fidelity enzyme was used in PCR, and the products were subjected to A-addition, adaptor ligation, and library pre-amplification experiments using the KAPA library construction kit. Chromosomal abnormalities were investigated. The results are shown in Table 11.

**Table 11 Detection of ploidy in intestinal cancer FFPE samples**

| Sample type | Sample No. | Primer 1: Ordinary primer | | | Primer 2: Primers with phosphorylated 5' end | | | Primer 3: Primers with G added to 5' end | | | Primer 4: Primers with G added to 5' end and phosphorylated | | |
|---|---|---|---|---|---|---|---|---|---|---|---|---|---|
| | | GAIN | LOSS | AI | GAIN | LOSS | AI | GAIN | LOSS | AI | GAIN | LOSS | AI |
| Intestinal cancer | 1 | / | / | / | / | / | / | / | / | / | 1q, | / | / |
| Intestinal cancer | 2 | / | / | / | / | 20p | / | / | 20p | 6q,11q | 7q | 20p | 6q,11q |
| Intestinal cancer | 3 | / | / | / | / | / | / | / | / | / | / | / | 1p,,5q, 8p,8q,1 1p,14q, 18p, |
| Intestinal cancer | 4 | 1q,7p,1 3q, | / | 1p,3q | 1q,7p,7 q,13q,2 0q | / | 1p,3q,5 p,5q,8p, 8q,11p, 11q,18q ,22q | 1q,7p,1 3q,20q | / | 1p,3q,1 1p,11q, 14q,18p ,18q,22 q | 1q,7p,7 q,13q,2 0q | / | 1p,3q,5 p,5q,8p ,8q,11p ,11q,14 q,18p,1 8q,22q |
| Intestinal cancer | 5 | / | / | 3q,5p ,8p,8 q, | / | / | 1p,3q,5 p,5q,8p, 8q, | / | / | 1p,3q,5 p,5q,8p, 8q, | / | / | 1p,3q,5 p,5q,8p ,8q |
| Intestinal cancer | 6 | / | / | / | / | 20p | / | / | 20p | / | / | 20p | / |
| Intestinal cancer | 7 | / | / | 1p,3q ,5p,5 q,8p, | 7q | / | 1p,3q,5 p,5q,8p, 8q 18q,2 2q | / | / | 1p,3q,5 p,5q,8p, 8q,,18q, | 7q | / | 1p,3q,5 p,5q,8p ,8q,,18 q,22q |
| Intestinal cancer | 8 | / | / | / | / | / | / | / | / | / | 1q,20q | / | / |
| Intestinal cancer | 9 | 7q | / | 11p,1 1q,18 p, | 7q | 20p | 1p,3q,5 p,11p,1 1q,14q, 18p, | 7q | / | 1p,3q,5 p,5q,8p, 8q,11p, 11q,18p , | 7q | 20p | 1p,3q,5 p,5q,8p ,8q,11p ,11q,14 q, 18p, |
| Intestinal cancer | 10 | / | / | / | / | / | / | / | / | / | / | / | / |
| Intestinal cancer | 11 | 7p,20q | / | 3q,5p ,8p,8 q, | 7p,20q | / | 1p,3q,5 q,8p,8q, 11q,14q | 7p,20q | / | 1p,3q,5 q,8p,8q, 11q,14q ,18p, | 7p,20q | / | 1p,3q,5 q,8p,8q ,11q,14 q,18p, |
| Intestinal cancer | 12 | / | / | 1p,3q ,5p, 8q,11 p,18p , | / | / | 1p,3q,5 p,8q,11 p,18p, | / | / | 1p,3q,5 p,5q,8p, 8q,11p, 18p, | / | / | 1p,3q,5 p,5q,8p ,8q,11p ,18p, |
| Intestinal cancer | 13 | / | / | / | / | / | / | / | / | / | / | / | 1p,3q,5 p,5q,8p ,8q |
| Intestinal cancer | 14 | 1q,7p | / | 6q,11 q,22q | 1q,7p | / | 18q,22q | 1q,7p | / | 6q,11q, 22q | 1q,7p | / | 6q,11q, 18q,22 9 |
| Intestinal cancer | 15 | / | / | / | / | / | / | / | / | / | / | / | / |
| Intestinal cancer | 16 | 13q | / | 1p,,5 q,18p , | 1q,13q | / | 1p,,5q,8 p,14q,1 8p, | 13q | / | 1p,,5q,1 1p,14q, 18p, | 1q,13q | / | 1p,,5q, 8p,8q,1 1p,14q, 18p, |
| Intestinal cancer | 17 | / | / | / | / | / | / | / | / | / | / | / | / |
| Intestinal cancer | 18 | / | / | / | 7q,13q | / | / | 7q, | / | / | 7q,13q | / | / |
| Intestinal cancer | 19 | 13q,20q | / | 5p,11 q,14q ,18p | 13q,20q | / | 5p,5q,1 1q,14q, 18p | 13q,20q | / | 5p,11p, 11q,14q ,18p | 13q,20q | / | 5p,5q,8 p,8q, 11 p,11q,1 4q,18p |
| Intestinal cancer | 20 | / | / | 3q,8p ,8q,1 1p,14 q | / | / | 3q,8p,8 q,11p,1 4q,18p, | / | / | 3q,8p,8 q,11p,1 4q | / | / | 3q,8p,8 q,11p,1 4q,18p, |
| Intestinal cancer | 21 | 1q,20q | / | 1p,3q ,5p,1 1q | 1q,7p,1 3q,20q | / | 1p,3q,5 p,11q,1 4q, | 1q,7p,2 0q | / | 1p,5p,1 1q,14q, 18p | 1q,7p,1 3q,20q | / | 1p,3q,5 p,11q,1 4q,18p, |
| Intestinal cancer | 22 | / | / | / | / | / | / | / | / | 6q,11q, 22q | 13q | / | / |
| Intestinal cancer | 23 | / | / | / | / | / | / | / | / | / | 13q,20q | / | / |
| Intestinal cancer | 24 | / | / | / | / | / | / | 1q,13q, 20q | / | / | 1q,13q, 20q | / | / |
| Intestinal cancer | 25 | / | / | / | / | / | / | / | 20p | / | / | 20p | / |

In the detection of intestinal cancer FFPE samples, the detection rate of chromosome ploidy changes in the case where amplicon library construction was performed using primer 1 was 11/25=44%; the detection rate of chromosome ploidy changes in the case where amplicon library construction was performed using primer 2 was 14/ 25=56%; the detection rate of chromosome ploidy changes in the case where amplicon library construction was performed using primer 3 was 17/25=68%; the detection rate of chromosome ploidy changes in the case where amplicon library construction was performed using primer 4 was 22/25=88 %; and more chromosomal abnormalities could be detected in the case where amplicon library construction was performed using primer 4.

### Example 13 Detection of ploidy in cervical exfoliated cell samples

Cervical exfoliated cell samples with clear pathological information were collected, including 15 cases of cervical cancer, 5 cases of CIN3, 5 cases of CIN2, and 5 cases of CIN1. These 30 samples were subjected to different methods to construct a ploidy library. The four kinds of primers in Sequence Listing 2 were used. Amplification was performed by QIAGEN multiplex enzyme (no need to add A to the product), and KAPA library construction kit was used for adaptor ligation and library pre-amplification. See Table 12 for detection results.

**Table 12 Detection of ploidy in cervical exfoliated cell samples**

| Sample type | Sample No. | Primer 1: Ordinary primer | | | Primer 2: Primers with phosphorylated 5' end | | | Primer 3: Primers with G added to 5' end | | | Primer 4: Primers with G added to 5' end and phosphorylated | | |
|---|---|---|---|---|---|---|---|---|---|---|---|---|---|
| | | GAIN | LOSS | AI | GAIN | LOSS | AI | GAIN | LOSS | AI | GAIN | LOSS | AI |
| Cervical cancer | 1 | 1q,16q | / | 16p | 1q,5p,9 q,16q | / | 3q,9p,1 6p | 1q,5p,9 q,16q | / | 3q,16p | 1q,5p,9 q,16q | / | 3q,9p,16p |
| Cervical cancer | 2 | / | / | 16p | 1q,16q | / | 3q,9p,1 6p | 1q,9q,1 6q | / | 3q,9p,1 6p | 1q,5p,9 q,16q | / | 3q,9p,16p |
| Cervical cancer | 3 | 3q,9p | 3p | / | 1q,3q,9 P | / | 4q | 1q,3q,9 P | 3p,4p | 4q | 1q,3q,9 P | 3p,4p | 4q |
| Cervical cancer | 4 | / | / | / | / | / | / | / | / | 2q,3q,1 0p,10q, 11p,13q | 2p,5p | 5q | 2q,3q,4q,6p ,6q,7p,7q,8 q,10p,10q,1 1p,13q |
| Cervical cancer | 5 | / | / | 4q,5p,1 1p,13q | 3q | / | 4p,7p, 1 1p,13q | 3q | / | 4q,5p,1 1p,13q | 3q | 3p | 4p,4q,5p,5q ,7p,11p,13q |
| Cervical cancer | 6 | / | / | 18q | 3q,8q | / | 18q | 3q,8q | / | 18q | 3q,8q | / | 18q |
| Cervical cancer | 7 | / | / | / | / | / | / | / | / | / | / | 4p,4q | / |
| Cervical cancer | 8 | / | / | / | / | / | / | 3q | / | 4p,5q,8 4 | 3q | / | 4p,5q,8q |
| Cervical cancer | 9 | / | / | / | / | / | 3q,4p,5 q,6p,8p, 8q,18p | / | / | 3q,4p,1 8p | / | / | 3q,4p,5q,6p ,8p,8q,18p |
| Cervical cancer | 10 | / | / | / | / | / | / | 1p,3q | / | 4q,17q | 1p,1q,2 p,3q | / | 4q,17q |
| Cervical cancer | 11 | / | / | / | 1p,1q,2 p,3q | / | 4q,16q, 17q | 1p,1q,2 p | / | 4q,16q | 1p,1q,2 p,3q | / | 4q,16q,17q |
| Cervical cancer | 12 | / | / | / | / | / | / | / | / | / | / | / | 3q,7p, lip |
| Cervical cancer | 13 | 1q,3q,8 P | 2q,4q | 4p,9q, 1 6q,18q | 1q,3q,8 q,9p,15 9 | 2q,4q | 4p,9q,1 1p,11q, 16q,18q | 1q,3q,8 p,15q | 2q,4q | 4p,9q,1 1p,11q, 16q,18q | 1q,3q,8 p,8q,9p, 15q | 2q,4q | 4p,9q,11p,1 1q,16q,18q |
| Cervical cancer | 14 | 1q,3q,1 5q | 2q | 4p,9q,1 1p,13q | 1q,3q,8 p,8q,9p | 2q,4q | 4p,9q,1 1p,11q, 18q | 1q,3q,9 p,15q | 2q | 4p,9q,1 1p,13q, 16q,18q | 1q,3q,8 p,8q,9p, 15q | 2q,4q | 4p,9q,11p,1 1q,13q,16q, 18q |
| Cervical cancer | 15 | / | / | 11p16q, 18q | / | / | 2q,4p,4 q,11q,1 5q,16q, 18q | 1q,3q,8 P | / | 2q,4p,4 q,9q,11 p16q,18 4 | 1q,3q,8 p,8q,9p | / | 2q,4p,4q,9q ,11p,11q,15 q,16q,18q |
| CIN3 | 16 | / | / | / | 3q | / | / | / | / | 3q,4q,6 q,8p,,11 q,18q | 3q | / | 4q,6q,8p,8q ,10q,11q,18 4 |
| CIN3 | 17 | / | / | / | / | / | / | 3q,5p | / | 4p,5q,6 p,8q,10 p,11q,1 8q | 3q,5p | / | 4p,4q,5q,6p ,6q,8p,8q,1 0p,10q,11q, 18q |
| CIN3 | 18 | / | / | / | 3q | / | / | / | / | / | 3q | / | / |
| CIN3 | 19 | / | / | / | / | / | / | / | / | / | / | / | / |
| CIN3 | 20 | / | / | / | / | / | / | 3q | / | / | 3q | / | / |
| CIN2 | 21 | / | / | / | / | / | 6p,6q | / | / | 6p,6q | / | / | 6p,6q |
| CIN2 | 22 | / | / | / | / | / | / | / | / | / | / | / | / |
| CIN2 | 23 | / | / | / | / | / | / | 3q | / | / | 3q,4q | / | / |
| CIN2 | 24 | / | / | / | / | / | / | / | / | / | / | / | / |
| CIN2 | 25 | / | / | / | / | / | / | / | / | / | 3q | / | / |
| CIN1 | 26 | / | / | / | / | / | / | / | / | / | / | / | / |
| CIN1 | 27 | / | / | / | / | / | / | / | / | / | / | / | / |
| CIN1 | 28 | / | / | / | / | / | / | / | / | / | / | / | / |
| CIN1 | 29 | / | / | / | / | / | / | / | / | / | / | / | / |
| CIN1 | 30 | / | / | / | / | / | / | / | / | / | / | / | / |

Results analysis: in the library of cervical cancer samples, the detection rate of primer 1 was 8/15=53.5%, and the detection rate of cervical cancer in the case where amplicon library construction was performed using primer 2 was 10/15=66.7%. The detection rate of primer 3 was 13/15=86.7%, and the detection rate of cervical cancer in the case where amplicon library construction was performed using primer 4 was 15/15=100%. In CIN3, the detection rate of primer 1 was 0/5=0%, the detection rate of primer 2 was 2/5=40%; the detection rate of primer 3 was 3/5=60%, and the detection rate of primer 4 was 4/5=80%.

In CIN2, the detection rate of primer 1 was 0/5=0%, the detection rate of primer 2 was 1/5=20%; the detection rate in the library constructed with primer 3 was 2/5=40%, and the detection rate of primer 4 was 3/5=60%. No sites were detected in the CIN1 sample using library constructed with four kinds of primers. Primer 4 (with G added at the 5' end in combination with phosphorylation) could detect more chromosomal abnormalities.

### Example 14 Detection of ploidy in urothelial cancer samples

20 cases of urothelial cancer samples with clear pathological information were collected, and subjected to different methods for ploidy library construction. The four kinds of primers in Table 18 were used. The PCR enzyme used was KAPA's multiplex enzyme (no need to add A to the product), and NEB library construction kit was used for adaptor ligation and library pre-amplification. The detection results are shown in Table 13.

**Table 13 Detection of loidv in urothelial cancer samples**

| Sample No. | Primer 1: Ordinary primer | | | Primer 2: Primers with phosphorylated 5' end | | | Primer 3: Primers with G added to 5' end | | | Primer 4: Primers with G added to 5' end and phosphorylated | | |
|---|---|---|---|---|---|---|---|---|---|---|---|---|
| | GAIN | LOSS | AI | GAIN | LOSS | AI | GAIN | LOSS | AI | GAIN | LOSS | AI |
| 1 | 1q | / | 4p,9q | 1q,7q,8 4 | / | 4p,9q,16 q,18p | 1q,7q | / | 4p,11q, 13 q,16q,18p | 1q,7q,8q, 17q,18q | / | 4p,9q,11p,1 1q,13q,16q, 18p |
| 2 | / | / | / | / | 4p | 4p,4q,5q , 11q,18q | / | 4p | 4p,4q,5q, 8p,8q,10p ,10q,11q, 18q | / | 4p | 4p,4q,5q,6p, 6q,8p,8q,10 p,10q,11q,1 8q |
| 3 | / | / | / | / | / | 4q,5q,6p ,6q, | / | / | 4p,4q,6p, 6q, | 7q,8q, | / | 4p,4q,5q,6p, 6q, |
| 4 | / | 11p,11q | / | / | / | / | / | 11p,11q | / | / | 11p,11q | / |
| 5 | 1q,17q | 11q, | / | 1 q, 17q, 18q | 11q,13q , | / | 1q,17q,18 q | 11q | / | 1q,17q, 18 4 | 11q,13q, | / |
| 6 | / | / | 4q,5q,6 p,6q | / | / | 4q,5q,6p | / | / | 4q,5q,6q | / | / | 4q,5q,6p,6q |
| 7 | / | / | / | / | 11q,13q , | / | 5q | / | / | / | 11q,13q, | / |
| 8 | 18q | / | / | 18q | / | / | 18q | / | / | 18q | / | / |
| 9 | 17q,18q | / | 4p,6p,6 q,8p,10 9 | 17q,18q | / | 4p,6q,8p ,18q | 17q,18q | / | 4p,6p,6q, 8p,10q,11 q,18q | 17q,18q | 11p,11q | 4p,6p,6q,8p, 10q,11q,18q |
| 10 | / | / | / | / | / | / | / | / | / | / | / | / |
| 11 | 17q | 11q,13q , | 1p,14q, 18p,18q ,22q | 17q | / | 1p,3q,5p ,5q,8p,8 q,11p,11 q,18q,22 q | 17q | 11q,13q, | 1p,3q,5p, 5q,8p,8q, 11p,14q,1 8q,22q | 17q | 11q,13q, | 1p,3q,5p,5q, 8p,8q,11p,1 1q,14q,18p, 18q,22q |
| 12 | / | / | / | / | / | / | / | / | / | / | 13q, | / |
| 13 | | / | 4q,5q,6 p,6q, | | / | / | / | / | 4q,5q,6p, 6q, | | 4p | 4q,5q,6p,6q, |
| 14 | / | / | / | 18q | / | / | 1q,17q,18 9 | / | / | 18q | / | / |
| 15 | 1q,18q | / | 1p,3q,5 p,14q18 q,22q | 1q,17q, 18q | / | 1p,3q,5p ,11q,18p ,18q,22q | 1q,18q | / | 1p,3q,5p, 11q,14q1 8q,22q | 1q,17q,18 4 | / | 1lp,3q,5p,11 q,14q,18p,1 8q,22q |
| 16 | / | / | / | / | / | / | / | / | / | / | / | / |
| 17 | / | / | / | / | / | / | / | / | / | / | 9q,13q, | / |
| 18 | 7q | / | 18p | 7q,8q, | / | 16q,18p | 7q | / | 16q,18p | 7q,8q, | / | 16q,18p |
| 19 | / | 11q | 4p,6p,6 q,8p, | / | 11q,13q , | 6q,8p,10 q,11q,18 q | / | 11q | 4p,6p,6q, 8p,10q, | / | 11q,13q, | 4p,6p,6q,8p, 10q,11q,18q |
| 20 | / | / | / | 17q | / | 16q | / | / | 16q,18q | 17q | / | 16q,18q |
| 21 | / | / | / | / | 8p,11q | 4p,6p,6q ,11q,18q | / | 8p,11q | 4p,8p,10q ,18q | / | 8p,11q | 4p,6p,6q,8p, 10q,11q,18q |
| 22 | 17q,18q | / | 8p,11q, 14q,18p ,18q | 17q,18q | / | 3q,5p,8p ,11q,14q ,18p,18q | 17q,18q | / | 3q,5p,11q ,14q,18p, 18q | 17q,18q | / | 3q,5p,8p,8q, 11p,11q,14q ,18p,18q |
| 23 | / | / | / | / | / | / | / | / | / | 7q, | / | / |
| 24 | / | 9q,13q, | 8q, | / | 9q,13q, | 8q,11p | / | 9q,13q, | 8q,11p | / | 9q,13q, | 8q,11p |
| 25 | 1q,17q, 18q | / | 5q,8p,8 q,11p | 1q,17q, 18q | / | 1p,5p,5q ,11p,11q ,18q,22q | 1q,17q,18 q | / | 5q,8p,11p ,11q,18q, 22q | 1q,17q, 18 4 | / | 1p,5p,5q,8p, 8q,11p,11q, 18q,22q |

Results analysis: in the library of urothelial cancer samples, the detection rate of chromosome ploidy changes in the case where amplicon library construction was performed using primer 1 was 14/25=56%; the detection rate of chromosome ploidy changes in the case where amplicon library construction was performed using primer 2 was 18/ 25=72%; the detection rate of chromosome ploidy changes in the case where amplicon library construction was performed using primer 3 was 21/25=84%; the detection rate of chromosome ploidy changes in the case where amplicon library construction was performed using primer 4 was 23/25=92%. The amplicon library construction using primer 4 (with G added at the 5' end in combination with phosphorylation) could lead more chromosomal abnormalities being detected.

### Example 15: RNA fusion detection

RNA fusion reference: Seraseq^{®} Fusion RNA Mix v4 were blended with wild-type (fusion-negative) cell line GM12878 RNA with confirmed genetic background at a concentration of 25 ng/µL in different volumes, and the lower detection limits of library construction method with four different modified primers in Table 19 were investigated. RNA was reverse transcribed using SuperScript^{™} VILO^{™} cDNA synthesis kit, and QIAGEN kit was used for cDNA purification. QIAGEN multiplex enzymes were used in multiplex PCR (no need to add A to the product), and Vazyme library construction kit was used for adaptor ligation and library pre-amplification. Each fusion type was tested 10 times at different blending ratios. The detection results are shown in Table 14.

**Table 14 Detection results of 4 kinds of primers**

| Blending concentration | Fusion type | Primer 1: Ordinary primer | Primer 2: modified with phosphorylated 5' end | Primer 3: with G added to 5' end | Primer 4: modified with G added to 5' end and phosphorylated |
|---|---|---|---|---|---|
| 10% | EML4-ALK | 30/30 | 30/30 | 30/30 | 30/30 |
| | CD74-ROS1 | 28/30 | 30/30 | 30/30 | 30/30 |
| | CCDC6-RET | 29/30 | 30/30 | 30/30 | 30/30 |
| | NCOA4-RET | 26/30 | 27/30 | 28/30 | 30/30 |
| | TPM3-NTRK1 | 28/30 | 29/30 | 30/30 | 30/30 |
| 5% | EML4-ALK | 26/30 | 27/30 | 28/30 | 30/30 |
| | CD74-ROS1 | 28/30 | 30/30 | 30/30 | 30/30 |
| | CCDC6-RET | 27/30 | 27/30 | 29/30 | 30/30 |
| | NCOA4-RET | 26/30 | 27/30 | 28/30 | 30/30 |
| | TPM3-NTRK1 | 24/30 | 26/30 | 28/30 | 30/30 |
| 2.5% | EML4-ALK | 22/30 | 24/30 | 26/30 | 30/30 |
| | CD74-ROS1 | 20/30 | 21/30 | 26/30 | 30/30 |
| | CCDC6-RET | 19/30 | 21/30 | 23/30 | 25/30 |
| | NCOA4-RET | 15/30 | 17/30 | 22/30 | 27/30 |
| | TPM3-NTRK1 | 16/30 | 18/30 | 21/30 | 26/30 |
| 1.25% | EML4-ALK | 15/30 | 20/30 | 24/30 | 26/30 |
| | CD74-ROS1 | 16/30 | 21/30 | 26/30 | 29/30 |
| | CCDC6-RET | 13/30 | 14/30 | 17/30 | 21/30 |
| | NCOA4-RET | 10/30 | 12/30 | 16/30 | 22/30 |
| | TPM3-NTRK1 | 10/30 | 12/30 | 18/30 | 24/30 |
| 0% | EML4-ALK | 0/30 | 0/30 | 0/30 | 0/30 |
| | CD74-ROS1 | 0/30 | 0/30 | 0/30 | 0/30 |
| | CCDC6-RET | 0/30 | 0/30 | 0/30 | 0/30 |
| | NCOA4-RET | 0/30 | 0/30 | 0/30 | 0/30 |
| | TPM3-NTRK1 | 0/30 | 0/30 | 0/30 | 0/30 |

Result analysis: Detection for five fusion types was performed. When the blending concentration was 0%, it was substantially undetectable for both methods. When the blending concentration was 10%, the cumulative detection rate of primer 1 was 94%. The cumulative detection rate of primer 2 was 97.3%, the cumulative detection rate of primer 3 was 98.7%, and the cumulative detection rate of primer 4 was 100%; in the case of 5% blending concentration, the cumulative detection rate of primer 1 was 87.3%, and the cumulative detection rate of primer 2 was 91.3 %, the cumulative detection rate of primer 3 was 95.3%, and the cumulative detection rate of primer 4 was 100%; in the case of 2.5% blending concentration, the cumulative detection rate of primer 1 was 61.3%, the cumulative detection rate of primer 2 was 67.3%, and the cumulative detection rate of primer 3 was 78.7%, and the cumulative detection rate of primer 4 was 92%; in the case of 1.25% blending concentration, the cumulative detection rate of primer 1 was 42.7%, the detection rate of primer 2 was 52.6%, the cumulative detection rate of primer 3 was 67.3%, and the cumulative detection rate of primer 4 was 81.3%. It could be seen from the above results that the effect of primer 4 was better than primer 1 when detecting low-frequency fusion.

### Example 16 Detection of pathogenic microorganisms

Amplicon regions to be detected were integrated into the same plasmid containing the T7 promoter. After performing in vitro transcription, an RNA reference was obtained. The size and concentration of the target fragment were detected by 2100, and then converted into a concentration in copy number. References with different copy numbers were used to evaluate the detection capabilities of amplicons for different modified primers. Reverse transcription was performed using SuperScript IV reverse transcriptase, and QIAGEN kit was used for cDNA purification. Multiplex PCR was performed by four kinds of primers in Table 20, respectively, KAPA multiplex enzyme was used in multiplex PCR (no need to add A to the product), and NEB library construction kit was used for adaptor ligation and library pre-amplification. Detection was performed 10 times for different copy numbers. The detection results are shown in Table 15.

**Table 15 Detection of 4 different primers**

| Initial copy number of reverse transcriptional reaction | Pathogenic microorganism type | Primer 1: Ordinary primer | Primer 2: modified with phosphorylated 5' end | Primer 3: with G added to 5' end | Primer 4: modified with G added to 5' end and phosphorylated |
|---|---|---|---|---|---|
| 10 | Influenza A virus | 10/10 | 10/10 | 10/10 | 10/10 |
| | influenza B virus | 10/10 | 10/10 | 10/10 | 10/10 |
| | covid-19 | 10/10 | 10/10 | 10/10 | 10/10 |
| 5 | Influenza A virus | 10/10 | 10/10 | 10/10 | 10/10 |
| | influenza B virus | 9/10 | 9/10 | 9/10 | 10/10 |
| | covid-19 | 9/10 | 10/10 | 10/10 | 10/10 |
| 2.5 | Influenza A virus | 7/10 | 8/10 | 9/10 | 10/10 |
| | influenza B virus | 6/10 | 7/10 | 8/10 | 9/10 |
| | covid-19 | 7/10 | 8/10 | 9/10 | 10/10 |
| 0 | Influenza A virus | 0/10 | 0/10 | 0/10 | 0/10 |
| | influenza B virus | 0/10 | 0/10 | 0/10 | 0/10 |
| | covid-19 | 0/10 | 0/10 | 0/10 | 0/10 |

Result analysis: the detection capabilities of three pathogenic microorganisms at different copy numbers (reverse transcription reaction) were examined: for 10 copies, it could be detected in all 10 tests; for 5 copies, the detection rate of primer 1 was 93.3%, the detection rate of primer 2 was 96.7%, the detection rate of primer 3 was 96.7%, the detection rate of primer 4 was 100%; for 2.5 copies, the detection rate of primer 1 was 66.7%, the detection rate of primer 2 was 76.7%, the detection rate of primer 3 was 86.7%, and the detection rate of primer 4 was 96.7%; for 0 copies, two primers were tested negative. Detection of low-concentration pathogenic microorganisms with primer 4 showed a better effect than primer 1.

### Example 17: Comparison of TA cloning efficiency of PCR products with different primers

Primers synthesized in different ways as shown in Table 21 were used, to amplify the promoter region of the purg gene of zebrafish. Three TA clones were tested in parallel for each primer type. PCR products were subjected to TA cloning according to the method in Example 1. The number of white spots (confirmation of insertion) and cloning efficiency (white spot rate) are detailed in Table 16 below.

**Table 16 Cloning efficiency of PCR products with different primers**

| Primer type used for amplification | Parallel 1 | | Parallel 2 | | Parallel 3 | | Total | |
|---|---|---|---|---|---|---|---|---|
| | Number of white spots | Cloning efficiency | Number of white spots | Cloning efficiency | Number of white spots | cloning efficiency | Number of white spots | Cloning efficiency |
| Ordinary primer | 415 | 46% | 408 | 44% | 426 | 47% | 1249 | 46% |
| Primers with phosphorylated 5' end | 505 | 58% | 512 | 56% | 509 | 59% | 1526 | 57.7% |
| Primers with G added to 5' end | 613 | 71% | 603 | 70% | 626 | 72% | 1842 | 71% |
| Primers with G added to 5' end and phosphorylated | 723 | 81% | 736 | 85% | 729 | 82% | 2188 | 82.7% |

Result analysis: ordinary primers had the worst effect on the number of white spots and cloning efficiency, and the primers with G added to 5' end and phosphorylated had the best effect. Because all other control conditions in the experiment (transfection, plating, culture conditions, etc.) were the same, and the single variable was different primers, it showed that products with primers with G added to 5' end and phosphorylated have higher TA ligation efficiency.

The primers used in each example were specifically as follows.

**Table 17 Mutation detection primers**

| No | Gene | Ch r | Ampl icon_ Start | Ampl icon_ Stop | 1: Ordinary primer | | 2: Primers with phosphorylated 5' end | | 3: Primers with G added to 5' end | | 4: Primers with G added to 5' end and phosphorylated | |
|---|---|---|---|---|---|---|---|---|---|---|---|---|
| | | | | | Fwd Primer | Rev Primer | Fwd Primer | Rev Primer | Fwd Primer | Rev Primer | Fwd Primer | Rev Primer |
| 1 | CTN NB1 | chr 3 | 4126 6108 | 4126 6224 | CAGTCTT ACCTGGA CTCTGGA ATC | CTCTTAC CAGCTAC TTGTTCT TGAGTG | CAGTCTT ACCTGGA CTCTGGA ATC | CTCTTACC AGCTACTT GTTCTTGA GTG | GCAGTCT TACCTGG ACTCTGG AATC | GCTCTTAC CAGCTACT TGTTCTTG AGTG | GCAGTCTT ACCTGGA CTCTGGAA TC | GCTCTTA CCAGCTA CTTGTTC TTGAGTG |
| 2 | DNA H2 | chr 17 | 7690 208 | 7690 307 | GTTTCCTC ATCACCC ACCATCT | GCCGTTG ATGAGGG TCAACA | GTTTCCTC ATCACCC ACCATCT | GCCGTTG ATGAGGG TCAACA | GTTTCCT CATCACC CACCATC T | GCCGTTGA TGAGGGTC AACA | GTTTCCTC ATCACCCA CCATCT | GCCGTTG ATGAGG GTCAACA |
| 3 | ERB B3 | chr 12 | 5647 8889 | 5647 9009 | CCAGGTC TACGATG GGAAGTT T | CATCATC AAGGAG GTACCAG TCTTG | CCAGGTC TACGATG GGAAGTT T | CATCATC AAGGAGG TACCAGT CTTG | GCCAGGT CTACGAT GGGAAGT TT | GCATCATC AAGGAGGT ACCAGTCT TG | GCCAGGT CTACGATG GGAAGTTT | GCATCAT CAAGGA GGTACCA GTCTTG |
| 4 | FBX W7 | chr 4 | 1532 4928 4 | 1532 4939 8 | ATTTAAG AGCACAC TGTCACT ATTTCAG T | GAGAATG TATACAC ACCTTAT ATGGGCA | ATTTAAG AGCACAC TGTCACT ATTTCAG T | GAGAATG TATACAC ACCTTATA TGGGCA | GATTTAA GAGCACA CTGTCAC TATTTCA GT | GAGAATGT ATACACAC CTTATATG GGCA | GATTTAA GAGCACA CTGTCACT ATTTCAGT | GAGAAT GTATACA CACCTTA TATGGGC A |
| 5 | PIK3 CA | chr 3 | 1789 3601 9 | 1789 3610 3 | ATTTTAC AGAGTAA CAGACTA GCTAGAG ACA | GCACTTA CCTGTGA CTCCATA GAAAAT | ATTTTAC AGAGTAA CAGACTA GCTAGAG ACA | GCACTTA CCTGTGA CTCCATA GAAAAT | GATTTTA CAGAGTA ACAGACT AGCTAGA GACA | GCACTTAC CTGTGACT CCATAGAA AAT | GATTTTAC AGAGTAA CAGACTA GCTAGAG ACA | GCACTTA CCTGTGA CTCCATA GAAAAT |
| 6 | PIK3 CA | chr 3 | 1789 3601 9 | 1789 3610 3 | ATTTTAC AGAGTAA CAGACTA GCTAGAG ACA | GCACTTA CCTGTGA CTCCATA GAAAAT | ATTTTAC AGAGTAA CAGACTA GCTAGAG ACA | GCACTTA CCTGTGA CTCCATA GAAAAT | GATTTTA CAGAGTA ACAGACT AGCTAGA GACA | GCACTTAC CTGTGACT CCATAGAA AAT | GATTTTAC AGAGTAA CAGACTA GCTAGAG ACA | GCACTTA CCTGTGA CTCCATA GAAAAT |
| 7 | PIK3 CA | chr 3 | 1789 3609 2 | 1789 3616 9 | CGAGATC CTCTCTCT GAAATCA CTGA | GCTGAGA TCAGCCA AATTCAG TTATTTTT | CGAGATC CTCTCTCT GAAATCA CTGA | GCTGAGA TCAGCCA AATTCAG TTATTTTT | GCGAGAT CCTCTCT CTGAAAT CACTGA | GCTGAGAT CAGCCAAA TTCAGTTA TTTTT | GCGAGAT CCTCTCTC TGAAATC ACTGA | GCTGAG ATCAGCC AAATTCA GTTATTT TT |
| 8 | PIK3 CA | chr 3 | 1789 1680 7 | 1789 1690 0 | CCCTCCA TCAACTT CTTCAAG ATGA | CCTACTG GTTCAAT TACTTTT AAAAAG GGT | CCCTCCA TCAACTT CTTCAAG ATGA | CCTACTG GTTCAATT ACTTTTAA AAAGGGT | GCCCTCC ATCAACT TCTTCAA GATGA | GCCTACTG GTTCAATT ACTTTTAA AAAGGGT | GCCCTCCA TCAACTTC TTCAAGAT GA | GCCTACT GGTTCAA TTACTTT TAAAAA GGGT |
| 9 | PIK3 CA | chr 3 | 1789 5206 2 | 1789 5218 5 | TGAGCAA GAGGCTT TGGAGTA TTTC | AGAGTTA TTAACAG TGCAGTG TGGAAT | TGAGCAA GAGGCTT TGGAGTA TTTC | AGAGTTA TTAACAG TGCAGTG TGGAAT | GTGAGCA AGAGGCT TTGGAGT ATTTC | GAGAGTTA TTAACAGT GCAGTGTG GAAT | GTGAGCA AGAGGCT TTGGAGTA TTTC | GAGAGT TATTAAC AGTGCA GTGTGGA AT |
| 10 | PIK3 CA | chr 3 | 1789 1740 6 | 1789 1751 2 | GTGATCT TCCAAAT CTACAGA GTTCCC | AGTCCTG TACTTCT GGATCTT TAACCAT | GTGATCT TCCAAAT CTACAGA GTTCCC | AGTCCTGT ACTTCTGG ATCTTTAA CCAT | GTGATCT TCCAAAT CTACAGA GTTCCC | GAGTCCTG TACTTCTG GATCTTTA ACCAT | GTGATCTT CCAAATCT ACAGAGT TCCC | GAGTCCT GTACTTC TGGATCT TTAACCA T |
| 11 | PPP2 R1A | chr 19 | 5271 5903 | 5271 6027 | CTTGCTCC TCTCTGCC ATACTG | GATCTCA CTCTTGA CGTTGTC CA | CTTGCTC CTCTCTG CCATACT G | GATCTCA CTCTTGAC GTTGTCCA | GCTTGCT CCTCTCT GCCATAC TG | GATCTCAC TCTTGACG TTGTCCA | GCTTGCTC CTCTCTGC CATACTG | GATCTCA CTCTTGA CGTTGTC CA |
| 12 | PTE N | chr 10 | 8969 2795 | 8969 2910 | GTTGCAC AATATCC TTTTGAA GACCAT | CCCGATG TAATAAA TATGCAC ATATCAT TACAC | GTTGCAC AATATCC TTTTGAA GACCAT | CCCGATG TAATAAA TATGCAC ATATCATT ACAC | GTTGCAC AATATCC TTTTGAA GACCAT | GCCCGATG TAATAAAT ATGCACAT ATCATTAC AC | GTTGCACA ATATCCTT TTGAAGA CCAT | GCCCGAT GTAATAA ATATGCA CATATCA TTACAC |
| 13 | PTE N | chr 10 | 8971 7567 | 8971 7693 | ATCGTTTT TGACAGT TTGACAG TTAAAGG | CGGCTGA GGGAACT CAAAGT | ATCGTTTT TGACAGT TTGACAG TTAAAGG | CGGCTGA GGGAACT CAAAGT | GATCGTT TTTGACA GTTTGAC AGTTAAA GG | GCGGCTGA GGGAACTC AAAGT | GATCGTTT TTGACAGT TTGACAGT TAAAGG | GCGGCT GAGGGA ACTCAAA GT |
| 14 | SMO | chr 7 | 1288 4633 5 | 1288 4645 1 | GGACTCT GTGAGTG GGATTTG T | CCCAGTA TATTTTG TTGCCCA ACTG | GGACTCT GTGAGTG GGATTTG T | CCCAGTA TATTTTGT TGCCCAA CTG | GGACTCT GTGAGTG GGATTTG T | GCCCAGTA TATTTTGT TGCCCAAC TG | GGACTCTG TGAGTGG GATTTGT | GCCCAGT ATATTTT GTTGCCC AACTG |
| 15 | TP53 | chr 17 | 7578 241 | 7578 366 | AAAGTGT TTCTGTCA TCCAAAT ACTCCA | CTGCTCA GATAGCG ATGGTGA | AAAGTGT TTCTGTC ATCCAAA TACTCCA | CTGCTCA GATAGCG ATGGTGA | GAAAGTG TTTCTGT CATCCAA ATACTCC A | GCTGCTCA GATAGCGA TGGTGA | GAAAGTG TTTCTGTC ATCCAAAT ACTCCA | GCTGCTC AGATAG CGATGGT GA |
| 16 | TP53 | chr 17 | 7577 508 | 7577 617 | GGCTCCT GACCTGG AGTCTT | CCTCATC TTGGGCC TGTGTT | GGCTCCT GACCTGG AGTCTT | CCTCATCT TGGGCCT GTGTT | GGCTCCT GACCTGG AGTCTT | GCCTCATC TTGGGCCT GTGTT | GGCTCCTG ACCTGGA GTCTT | GCCTCAT CTTGGGC CTGTGTT |
| 17 | TP53 | chr 17 | 7577 031 | 7577 157 | CTTGCTTA CCTCGCTT AGTGCT | CTTGCTT CTCTTTT CCTATCC TGAGT | CTTGCTT ACCTCGC TTAGTGC T | CTTGCTTC TCTTTTCC TATCCTGA GT | GCTTGCT TACCTCG CTTAGTG CT | GCTTGCTT CTCTTTTC CTATCCTG AGT | GCTTGCTT ACCTCGCT TAGTGCT | GCTTGCT TCTCTTT TCCTATC CTGAGT |
| 18 | TP53 | chr 17 | 7577 031 | 7577 157 | CTTGCTTA CCTCGCTT AGTGCT | CTTGCTT CTCTTTT CCTATCC TGAGT | CTTGCTT ACCTCGC TTAGTGC T | CTTGCTTC TCTTTTCC TATCCTGA GT | GCTTGCT TACCTCG CTTAGTG CT | GCTTGCTT CTCTTTTC CTATCCTG AGT | GCTTGCTT ACCTCGCT TAGTGCT | GCTTGCT TCTCTTT TCCTATC CTGAGT |
| 19 | TP53 | chr 17 | 7577 031 | 7577 157 | CTTGCTTA CCTCGCTT AGTGCT | CTTGCTT CTCTTTT CCTATCC TGAGT | CTTGCTT ACCTCGC TTAGTGC T | CTTGCTTC TCTTTTCC TATCCTGA GT | GCTTGCT TACCTCG CTTAGTG CT | GCTTGCTT CTCTTTTC CTATCCTG AGT | GCTTGCTT ACCTCGCT TAGTGCT | GCTTGCT TCTCTTT TCCTATC CTGAGT |
| 20 | KRA S | chr 12 | 2539 8119 | 2539 8270 | TAAGTAC TCATGAA AATGGTC AGAGAAA C | AGTGCCT TGACGAT ACAGCTA ATTC | TAAGTAC TCATGAA AATGGTC AGAGAAA C | AGTGCCTT GACGATA CAGCTAA TTC | GTAAGTA CTCATGA AAATGGT CAGAGAA AC | GAGTGCCT TGACGATA CAGCTAAT TC | GTAAGTA CTCATGAA AATGGTC AGAGAAA C | GAGTGC CTTGACG ATACAGC TAATTC |
| 21 | NRA S | chr 1 | 1152 5861 4 | 1152 5883 5 | AGATGAT CCGACAA GTGAGAG ACA | GGCTCGC CAATTAA CCCTGA | AGATGAT CCGACAA GTGAGAG ACA | GGCTCGC CAATTAA CCCTGA | GAGATGA TCCGACA AGTGAGA GACA | GGCTCGCC AATTAACC CTGA | GAGATGA TCCGACA AGTGAGA GACA | GGCTCGC CAATTAA CCCTGA |
| 22 | ASX L1 | chr 20 | 3102 5073 | 3102 5256 | GGTGCGT TCTGTCA CGATGA | AGAGTGC TCCTGCC TAAAGAG T | GGTGCGT TCTGTCA CGATGA | AGAGTGC TCCTGCCT AAAGAGT | GGTGCGT TCTGTCA CGATGA | GAGAGTGC TCCTGCCT AAAGAGT | GGTGCGTT CTGTCACG ATGA | GAGAGT GCTCCTG CCTAAAG AGT |
| 23 | DN MT3 A | chr 2 | 2546 8850 | 2546 9065 | CACACTA GGAGTGC CAGAGTT | CCCAAGG TCAAGGA GATTATT GATGAG | CACACTA GGAGTGC CAGAGTT | CCCAAGG TCAAGGA GATTATTG ATGAG | GCACACT AGGAGTG CCAGAGT T | GCCCAAGG TCAAGGAG ATTATTGA TGAG | GCACACT AGGAGTG CCAGAGTT | GCCCAA GGTCAA GGAGATT ATTGATG AG |
| 24 | IDH2 | chr 15 | 9063 1714 | 9063 1905 | CACAAAG TCTGTGG CCTTGTA CT | GAGCCCA TCATCTG CAAAAAC ATC | CACAAAG TCTGTGG CCTTGTA CT | GAGCCCA TCATCTGC AAAAACA TC | GCACAAA GTCTGTG GCCTTGT ACT | GAGCCCAT CATCTGCA AAAACATC | GCACAAA GTCTGTGG CCTTGTAC T | GAGCCC ATCATCT GCAAAA ACATC |
| 25 | DN MT3 A | chr 2 | 2546 9546 | 2546 9691 | CAATCAT GGGCTTG TTCTGCA C | TTCCAGC CTGTCCT GACAAC | CAATCAT GGGCTTG TTCTGCA C | TTCCAGCC TGTCCTGA CAAC | GCAATCA TGGGCTT GTTCTGC AC | GTTCCAGC CTGTCCTG ACAAC | GCAATCA TGGGCTTG TTCTGCAC | GTTCCAG CCTGTCC TGACAAC |
| 26 | TET 2 | chr 4 | 1061 5800 0 | 1061 5821 0 | ACCCCAA ACTGAGT CTTGCCA TA | GGCGTGA AACTGCT TCAGATG | ACCCCAA ACTGAGT CTTGCCA TA | GGCGTGA AACTGCTT CAGATG | GACCCCA AACTGAG TCTTGCC ATA | GGCGTGAA ACTGCTTC AGATG | GACCCCA AACTGAG TCTTGCCA TA | GGCGTG AAACTGC TTCAGAT G |
| 27 | PTP N11 | chr 12 | 1129 2673 2 | 1129 2686 5 | TTAGCAT TGTCTCTG AGTCCAC TAAAA | CTGGATG GTTTTGG GAACGTC AATA | TTAGCAT TGTCTCT GAGTCCA CTAAAA | CTGGATG GTTTTGGG AACGTCA ATA | GTTAGCA TTGTCTC TGAGTCC ACTAAAA | GCTGGATG GTTTTGGG AACGTCAA TA | GTTAGCAT TGTCTCTG AGTCCACT AAAA | GCTGGAT GGTTTTG GGAACG TCAATA |
| 28 | RUN X1 | chr 21 | 3617 1738 | 3617 1891 | GATGGTT GGATCTG CCTTGTAT CTG | ATTAAAC CCTGGTA CATAGGC CACATA | GATGGTT GGATCTG CCTTGTA TCTG | ATTAAAC CCTGGTA CATAGGC CACATA | GATGGTT GGATCTG CCTTGTA TCTG | GATTAAAC CCTGGTAC ATAGGCCA CATA | GATGGTTG GATCTGCC TTGTATCT G | GATTAA ACCCTGG TACATAG GCCACAT A |
| 29 | EGF R | chr 7 | 5524 9000 | 5524 9131 | CTCCAGG AAGCCTA CGTGATG | CAGTTGA GCAGGTA CTGGGAG | CTCCAGG AAGCCTA CGTGATG | CAGTTGA GCAGGTA CTGGGAG | GCTCCAG GAAGCCT ACGTGAT G | GCAGTTGA GCAGGTAC TGGGAG | GCTCCAG GAAGCCT ACGTGAT G | GCAGTTG AGCAGG TACTGGG AG |
| 30 | KRA S | chr 12 | 2539 8274 | 2539 8385 | GCTGTAT CGTCAAG GCACTCT TG | AGGTACT GGTGGAG TATTTGA TAGTGTA TT | GCTGTAT CGTCAAG GCACTCT TG | AGGTACT GGTGGAG TATTTGAT AGTGTATT | GCTGTAT CGTCAAG GCACTCT TG | GAGGTACT GGTGGAGT ATTTGATA GTGTATT | GCTGTATC GTCAAGG CACTCTTG | GAGGTA CTGGTGG AGTATTT GATAGTG TATT |
| 31 | CDK N2A | chr 9 | 2196 8163 | 2196 8300 | CTGTAGG ACCTTCG GTGACTG | TGTGCCA CACATCT TTGACC | CTGTAGG ACCTTCG GTGACTG | TGTGCCA CACATCTT TGACC | GCTGTAG GACCTTC GGTGACT G | GTGTGCCA CACATCTT TGACC | GCTGTAG GACCTTCG GTGACTG | GTGTGCC ACACATC TTTGACC |
| 32 | ERB B2 | chr 17 | 3786 8148 | 3786 8263 | TGTTCCAT CCTCTGCT GTCA | GGGTATG TGGCTAC ATGTTCC T | TGTTCCA TCCTCTG CTGTCA | GGGTATG TGGCTAC ATGTTCCT | GTGTTCC ATCCTCT GCTGTCA | GGGTATGT GGCTACAT GTTCCT | GTGTTCCA TCCTCTGC TGTCA | GGGTATG TGGCTAC ATGTTCC T |
| 33 | FGF R3 | chr 4 | 1803 545 | 1803 665 | GTCATCT GCCCCCA CAGA | TGCGTCA CTGTACA CCTTGC | GTCATCT GCCCCCA CAGA | TGCGTCA CTGTACA CCTTGC | GTCATCT GCCCCCA CAGA | GTGCGTCA CTGTACAC CTTGC | GTCATCTG CCCCCACA GA | GTGCGTC ACTGTAC ACCTTGC |
| 34 | HRA S | chr 11 | 5338 04 | 5339 26 | GTGGTCA TTGATGG GGAGAC | GATGGCA AACACAC ACAGGA | GTGGTCA TTGATGG GGAGAC | GATGGCA AACACAC ACAGGA | GTGGTCA TTGATGG GGAGAC | GATGGCAA ACACACAC AGGA | GTGGTCAT TGATGGG GAGAC | GATGGC AAACAC ACACAG GA |
| 35 | KRA S | chr 12 | 2537 8532 | 2537 8655 | TTTCAGT GTTACTT ACCTGTC TTGTCTT | GGAAATA AATGTGA TTTGCCT TCT | TTTCAGT GTTACTT ACCTGTC TTGTCTT | GGAAATA AATGTGA TTTGCCTT CT | GTTTCAG TGTTACT TACCTGT CTTGTCT T | GGAAATAA ATGTGATT TGCCTTCT | GTTTCAGT GTTACTTA CCTGTCTT GTCTT | GGAAAT AAATGTG ATTTGCC TTCT |
| 36 | MET | chr 7 | 1164 2337 8 | 1164 2350 1 | TTTTGAGT TTGCAGA CTTTCCA | TCAAGGT TGCTGAT TTTGGTC | TTTTGAG TTTGCAG ACTTTCC A | TCAAGGT TGCTGATT TTGGTC | GTTTTGA GTTTGCA GACTTTC CA | GTCAAGGT TGCTGATT TTGGTC | GTTTTGAG TTTGCAGA CTTTCCA | GTCAAG GTTGCTG ATTTTGG TC |
| 37 | MLL | chr 11 | 1183 5934 1 | 1183 5946 1 | GTTGCCTT CCACAAA CGTG | CAAGTCT GTTGTGA GCCCTTC | GTTGCCT TCCACAA ACGTG | CAAGTCT GTTGTGA GCCCTTC | GTTGCCT TCCACAA ACGTG | GCAAGTCT GTTGTGAG CCCTTC | GTTGCCTT CCACAAA CGTG | GCAAGT CTGTTGT GAGCCCT TC |
| 38 | PIK3 CA | chr 3 | 1789 1678 1 | 1789 1689 9 | GAAAAAG CCGAAGG TCACAA | CCCCCTC CATCAAC TTCTTC | GAAAAAG CCGAAGG TCACAA | CCCCCTCC ATCAACTT CTTC | GAAAAAG CCGAAGG TCACAA | GCCCCCTC CATCAACT TCTTC | GAAAAAG CCGAAGG TCACAA | GCCCCCT CCATCAA CTTCTTC |
| 39 | TP53 | chr 17 | 7572 893 | 7573 009 | GAGGCTG TCAGTGG GGAAC | GCCACCT GAAGTCC AAAAAG | GAGGCTG TCAGTGG GGAAC | GCCACCT GAAGTCC AAAAAG | GAGGCTG TCAGTGG GGAAC | GCCACCTG AAGTCCAA AAAG | GAGGCTG TCAGTGG GGAAC | GCCACCT GAAGTCC AAAAAG |
| 40 | VHL | chr 3 | 1018 3506 | 1018 3620 | CCGCCGT CTTCTTCA GG | CCCGGGT GGTCTGG AT | CCGCCGT CTTCTTCA GG | CCCGGGT GGTCTGG AT | GCCGCCG TCTTCTTC AGG | GCCCGGGT GGTCTGGA T | GCCGCCG TCTTCTTC AGG | GCCCGG GTGGTCT GGAT |
| 41 | TER T | chr 5 | 1295 189 | 1295 314 | GGCCGCG GAAAGGA AG | CGTCCTG CCCCTTC ACC | GGCCGCG GAAAGGA AG | CGTCCTGC CCCTTCAC C | GGCCGCG GAAAGGA AG | GCGTCCTG CCCCTTCA CC | GGCCGCG GAAAGGA AG | GCGTCCT GCCCCTT CACC |
| 42 | TP53 | chr 17 | 7577 068 | 7577 303 | GCGGAGA TTCTCTTC CTCTGTG | GGCTTCT CCTCCAC CTACCT | GCGGAGA TTCTCTTC CTCTGTG | GGCTTCTC CTCCACCT ACCT | GCGGAGA TTCTCTTC CTCTGTG | GGCTTCTC CTCCACCT ACCT | GCGGAGA TTCTCTTC CTCTGTG | GGCTTCT CCTCCAC CTACCT |
| 43 | TP53 | chr 17 | 7576 860 | 7577 121 | GTGAAAT ATTCTCC ATCCAGT GGTTTCT | CGTGTTT GTGCCTG TCCTG | GTGAAAT ATTCTCC ATCCAGT GGTTTCT | CGTGTTTG TGCCTGTC CTG | GTGAAAT ATTCTCC ATCCAGT GGTTTCT | GCGTGTTT GTGCCTGT CCTG | GTGAAAT ATTCTCCA TCCAGTGG TTTCT | GCGTGTT TGTGCCT GTCCTG |
| 44 | APC | Ch r5 | 1121 7546 8 | 1121 7573 7 | CTTGATA GTTTTGA GAGTCGT TCGATTG | AAGAACC TGGACCC TCTGAAC T | CTTGATA GTTTTGA GAGTCGT TCGATTG | AAGAACC TGGACCC TCTGAACT | GCTTGAT AGTTTTG AGAGTCG TTCGATT G | GAAGAAC CTGGACCC TCTGAACT | GCTTGATA GTTTTGAG AGTCGTTC GATTG | GAAGAA CCTGGAC CCTCTGA ACT |
| 45 | APC | Ch r5 | 1121 7380 2 | 1121 7405 5 | AAGAGGA AGCTTAG ATAGTTC TCGTTCT | GTGCATT TCTCTCA TCTGTCA CAC | AAGAGGA AGCTTAG ATAGTTC TCGTTCT | GTGCATTT CTCTCATC TGTCACA C | GAAGAG GAAGCTT AGATAGT TCTCGTT CT | GTGCATTT CTCTCATC TGTCACAC | GAAGAGG AAGCTTA GATAGTTC TCGTTCT | GTGCATT TCTCTCA TCTGTCA CAC |

**Table 18 Primers for ploidy detection**

| Primer | First primer : Ordinary primer | First primer: modified with phosphorylated 5' end | Third primer: with G added to 5' end | Fourth primer: modified with G added to 5' end and phosphorylated |
|---|---|---|---|---|
| F | NNNNNNNNNNNNNNNNNACA CAGGGAGGGGAACAT | NNNNNNNNNNNNNNNNNACAC AGGGAGGGGAACAT | GNNNNNNNNNNNNNNA CACAGGGAGGGAACAT | GNNNNNNNNNNNNNNNNNNACA CAGGGAGGGGAACAT |
| R | TGCCATGGTGGTTTGCT | TGCCATGGTGGTTTGCT | GTGCCATGGTGGTTTGCT | GTGCCATGGTGGTTTGCT |

| | | | | |
|---|---|---|---|---|
| Note: N represents random primer. | | | | |

**Table 19 Primers for fusion detection**

| Fusion type | Pr im er | Ordinary primer | | Modified with phosphorylated 5' end | | With G added to 5' end | | Modified with G added to 5' end and phosphorylated | |
|---|---|---|---|---|---|---|---|---|---|
| | | F | R | F | R | F | R | F | R |
| EML4-ALK | 1 | GAGCAAAAC TACTGTAGA GCCCACA | CATCTGCAT GGCTTGCAG CTCCTGGTG C | GAGCAAAAC TACTGTAGA GCCCACA | CATCTGCATG GCTTGCAGCT CCTGGTGC | GAGCAAAAC TACTGTAGA GCCCACA | GCATCTGCAT GGCTTGCAGC TCCTGGTGC | GAGCAAAA CTACTGTA GAGCCCAC A | GCATCTGC ATGGCTTG CAGCTCCT GGTGC |
| | 2 | GTCCCCAGA CAACAAGTA TATAATGT | TTGCAGCTC CTGGTGCTT CCGGCGGTA C | GTCCCCAGA CAACAAGTA TATAATGT | TTGCAGCTCC TGGTGCTTCC GGCGGTAC | GTCCCCAGA CAACAAGTA TATAATGT | GTTGCAGCTC CTGGTGCTTC CGGCGGTAC | GTCCCCAG ACAACAAG TATATAAT GT | GTTGCAGC TCCTGGTG CTTCCGGC GGTAC |
| | 3 | CATAAAGAT GTCATCATC AACCAAG | CTTGCCAGC AAAGCAGTA GTTGGGG | CATAAAGAT GTCATCATC AACCAAG | CTTGCCAGCA AAGCAGTAGT TGGGG | GCATAAAGA TGTCATCATC AACCAAG | GCTTGCCAGC AAAGCAGTA GTTGGGG | GCATAAAG ATGTCATC ATCAACCA AG | GCTTGCCA GCAAAGCA GTAGTTGG GG |
| | 4 | AGGAACATT TAATGATGG CTTCCAAAT AG | TACTCAGGG CTCTGCAGC TCCATCTG | AGGAACATT TAATGATGG CTTCCAAAT AG | TACTCAGGGC TCTGCAGCTC CATCTG | GAGGAACAT TTAATGATG GCTTCCAAA TAG | GTACTCAGG GCTCTGCAGC TCCATCTG | GAGGAAC ATTTAATG ATGGCTTC CAAATAG | GTACTCAG GGCTCTGC AGCTCCAT CTG |
| | 5 | TTTCTATCCA CACAGACGG GAATG | TCAGCTTGT ACTCAGGGC TCTGCAGC | TTTCTATCCA CACAGACGG GAATG | TCAGCTTGTA CTCAGGGCTC TGCAGC | GTTTCTATCC ACACAGACG GGAATG | GTCAGCTTGT ACTCAGGGCT CTGCAGC | GTTTCTAT CCACACAG ACGGGAAT G | GTCAGCTT GTACTCAG GGCTCTGC AGC |
| | 6 | ATCATGTGG CCTCAGTGA AAAAATC | GCTCCTGGT GCTTCCGGC GGTA | ATCATGTGG CCTCAGTGA AAAAATC | GCTCCTGGTG CTTCCGGCGG TA | GATCATGTG GCCTCAGTG AAAAAATC | GCTCCTGGTG CTTCCGGCGG TA | GATCATGT GGCCTCAG TGAAAAAA TC | GCTCCTGG TGCTTCCG GCGGTA |
| | 7 | CTGTGATGC GCTACTCAA TAG | TCCTGGTGC TTCCGGCGG T | CTGTGATGC GCTACTCAA TAG | TCCTGGTGCT TCCGGCGGT | GCTGTGATG CGCTACTCA ATAG | GTCCTGGTGC TTCCGGCGGT | GCTGTGAT GCGCTACT CAATAG | GTCCTGGT GCTTCCGG CGGT |
| | 8 | CCTCAGTGA AAAAATCAG TCTCAAG | GGCTTGCAG CTCCTGGTG | CCTCAGTGA AAAAATCAG TCTCAAG | GGCTTGCAGC TCCTGGTG | GCCTCAGTG AAAAAATCA GTCTCAAG | GGCTTGCAGC TCCTGGTG | GCCTCAGT GAAAAAAT CAGTCTCA AG | GGCTTGCA GCTCCTGG TG |
| | 9 | TTAATGATG GCTTCCAAA TAGAAGTA | GGCTTGCAG CTCCTGGTG CTTCCG | TTAATGATG GCTTCCAAA TAGAAGTA | GGCTTGCAGC TCCTGGTGCT TCCG | GTTAATGAT GGCTTCCAA ATAGAAGTA | GGCTTGCAGC TCCTGGTGCT TCCG | GTTAATGA TGGCTTCC AAATAGAA GTA | GGCTTGCA GCTCCTGG TGCTTCCG |
| | 10 | CCCACACCT GGGAAAGGA CCTA | ATCTGCATG GCTTGCAGC TC | CCCACACCT GGGAAAGGA CCTA | ATCTGCATGG CTTGCAGCTC | GCCCACACC TGGGAAAGG ACCTA | GATCTGCATG GCTTGCAGCT C | GCCCACAC CTGGGAAA GGACCTA | GATCTGCA TGGCTTGC AGCTC |
| | 11 | CTGAATCCT GAAAGAGAA ATAGAG | TGCATGGCT TGCAGCTCC TGGTG | CTGAATCCT GAAAGAGAA ATAGAG | TGCATGGCTT GCAGCTCCTG GTG | GCTGAATCC TGAAAGAGA AATAGAG | GTGCATGGCT TGCAGCTCCT GGTG | GCTGAATC CTGAAAGA GAAATAGA G | GTGCATGG CTTGCAGC TCCTGGTG |
| NPM1-ALK | 12 | CCAGTGCAT ATTAGTGGA CAGCA | GCAGCTCCT GGTGCTTCC GG | CCAGTGCAT ATTAGTGGA CAGCA | GCAGCTCCTG GTGCTTCCGG | GCCAGTGCA TATTAGTGG ACAGCA | GCAGCTCCTG GTGCTTCCGG | GCCAGTGC ATATTAGT GGACAGCA | GCAGCTCC TGGTGCTT CCGG |
| CLTC-ALK | 13 | AAGGAGTAC TTGACAAAG | GCATGGCTT GCAGCTCCT GGTGCTT | AAGGAGTAC TTGACAAAG | GCATGGCTTG CAGCTCCTGG TGCTT | GAAGGAGTA CTTGACAAA G | GCATGGCTTG CAGCTCCTGG TGCTT | GAAGGAGT ACTTGACA AAG | GCATGGCT TGCAGCTC CTGGTGCT T |
| | 14 | AAGAAGAAC AAGCTACAG AGACACAAC CCAT | TCTGCAGCT CCATCTGCA TGGC | AAGAAGAAC AAGCTACAG AGACACAAC CCAT | TCTGCAGCTC CATCTGCATG GC | GAAGAAGAA CAAGCTACA GAGACACAA CCCAT | GTCTGCAGCT CCATCTGCAT GGC | GAAGAAG AACAAGCT ACAGAGAC ACAACCCA T | GTCTGCAG CTCCATCT GCATGGC |
| CCDC 6-RET | 15 | CGCAAAGCA AAGCCAGCG TGACCATC | TCTTCACGG CCACCGTGG TGTACCCTG C | CGCAAAGCA AAGCCAGCG TGACCATC | TCTTCACGGC CACCGTGGTG TACCCTGC | GCGCAAAGC AAAGCCAGC GTGACCATC | GTCTTCACGG CCACCGTGGT GTACCCTGC | GCGCAAAG CAAAGCCA GCGTGACC ATC | GTCTTCAC GGCCACCG TGGTGTAC CCTGC |
| | 16 | AAGAATTCC TCACTAATG AGCTCTCCA G | TGGTGTACC CTGCTCTGC CTTTCAGAT | AAGAATTCC TCACTAATG AGCTCTCCA G | TGGTGTACCC TGCTCTGCCT TTCAGAT | GAAGAATTC CTCACTAAT GAGCTCTCC AG | GTGGTGTACC CTGCTCTGCC TTTCAGAT | GAAGAATT CCTCACTA ATGAGCTC TCCAG | GTGGTGTA CCCTGCTC TGCCTTTC AGAT |
| | 17 | GCAGCACAT GGGAACATC CCATGGTAT C | TCCACGGAG ACCTGGTTC TCCATGGAG TC | GCAGCACAT GGGAACATC CCATGGTAT C | TCCACGGAGA CCTGGTTCTC CATGGAGTC | GCAGCACAT GGGAACATC CCATGGTAT C | GTCCACGGA GACCTGGTTC TCCATGGAGT C | GCAGCACA TGGGAACA TCCCATGG TATC | GTCCACGG AGACCTGG TTCTCCAT GGAGTC |
| NCOA 4-RET | 18 | GCTTACCCA AAAGCAGAC CTTGGAGAA C | TCACGGCCA CCGTGGTGT ACCCTGCTC TG | GCTTACCCA AAAGCAGAC CTTGGAGAA C | TCACGGCCAC CGTGGTGTAC CCTGCTCTG | GCTTACCCA AAAGCAGAC CTTGGAGAA C | GTCACGGCC ACCGTGGTGT ACCCTGCTCT G | GCTTACCC AAAAGCAG ACCTTGGA GAAC | GTCACGGC CACCGTGG TGTACCCT GCTCTG |
| | 19 | GTGGATTCT AGTAGTGTG GATTCTAGT AGTTTATAT ACC | TGAGGGCAA ATGTTGATG TCTTGGGTCT | GTGGATTCT AGTAGTGTG GATTCTAGT AGTTTATAT ACC | TGAGGGCAAA TGTTGATGTC TTGGGTCT | GTGGATTCT AGTAGTGTG GATTCTAGT AGTTTATAT ACC | GTGAGGGCA AATGTTGATG TCTTGGGTCT | GTGGATTC TAGTAGTG TGGATTCT AGTAGTTT ATATACC | GTGAGGGC AAATGTTG ATGTCTTG GGTCT |
| KIF5B-RET | 20 | GAATTGCTG TGGGAAATA ATGATGT | GCCACCGTG GTGTACCCT GCTCTGC | GAATTGCTG TGGGAAATA ATGATGT | GCCACCGTGG TGTACCCTGC TCTGC | GAATTGCTG TGGGAAATA ATGATGT | GCCACCGTGG TGTACCCTGC TCTGC | GAATTGCT GTGGGAAA TAATGATG T | GCCACCGT GGTGTACC CTGCTCTG C |
| | 21 | GAGTTAGCA GCATGTCAG CTTCGTATCT CT | TCTTCACGG CCACCGTGG TGTACCCT | GAGTTAGCA GCATGTCAG CTTCGTATCT CT | TCTTCACGGC CACCGTGGTG TACCCT | GAGTTAGCA GCATGTCAG CTTCGTATCT CT | GTCTTCACGG CCACCGTGGT GTACCCT | GAGTTAGC AGCATGTC AGCTTCGT ATCTCT | GTCTTCAC GGCCACCG TGGTGTAC CCT |
| | 22 | TTCAGGACC TGGCTACAA GAGTTAA | TGGTGTACC CTGCTCTGC CTTTCAGAT | TTCAGGACC TGGCTACAA GAGTTAA | TGGTGTACCC TGCTCTGCCT TTCAGAT | GTTCAGGAC CTGGCTACA AGAGTTAA | GTGGTGTACC CTGCTCTGCC TTTCAGAT | GTTCAGGA CCTGGCTA CAAGAGTT AA | GTGGTGTA CCCTGCTC TGCCTTTC AGAT |
| CD74-ROS 1 | 23 | CCCACCCAC TGACGCTCC ACCGAA | CTCAAGGAT ATAGTATGT AATTCTACA T | CCCACCCAC TGACGCTCC ACCGAA | CTCAAGGATA TAGTATGTAA TTCTACAT | GCCCACCCA CTGACGCTC CACCGAA | GCTCAAGGA TATAGTATGT AATTCTACAT | GCCCACCC ACTGACGC TCCACCGA A | GCTCAAGG ATATAGTA TGTAATTC TACAT |
| | 24 | AGCAGGCAC TCCTTGGAG CAAAAGCCC | TGTAACAAC CAGAAATAT TCCAACTA | AGCAGGCAC TCCTTGGAG CAAAAGCCC | TGTAACAACC AGAAATATTC CAACTA | GAGCAGGCA CTCCTTGGA GCAAAAGCC C | GTGTAACAA CCAGAAATAT TCCAACTA | GAGCAGGC ACTCCTTG GAGCAAAA GCCC | GTGTAACA ACCAGAAA TATTCCAA CTA |
| SLC34 A2-ROS 1 | 25 | GTAGCGCCT TCCAGCTGG TTGGA | AAGGATATA GTATGTAAT TCTACATCC A | GTAGCGCCT TCCAGCTGG TTGGA | AAGGATATAG TATGTAATTC TACATCCA | GTAGCGCCT TCCAGCTGG TTGGA | GAAGGATAT AGTATGTAAT TCTACATCCA | GTAGCGCC TTCCAGCT GGTTGGA | GAAGGATA TAGTATGT AATTCTAC ATCCA |
| | 26 | ACAGGCGTG AGCCACCAC CAGGCCTGA | GGATATAGT ATGTAATTC TACA | ACAGGCGTG AGCCACCAC CAGGCCTGA | GGATATAGTA TGTAATTCTA CA | GACAGGCGT GAGCCACCA CCAGGCCTG A | GGATATAGTA TGTAATTCTA CA | GACAGGCG TGAGCCAC CACCAGGC CTGA | GGATATAG TATGTAAT TCTACA |
| TPM3-NTRK 1 | 27 | CTGGAAAAG ACAATTGAT GACCTGG | CCAGGTCAT CAATTGTCTT TTCCAG | CTGGAAAAG ACAATTGAT GACCTGG | CCAGGTCATC AATTGTCTTTT CCAG | GCTGGAAAA GACAATTGA TGACCTGG | GCCAGGTCAT CAATTGTCTT TTCCAG | GCTGGAAA AGACAATT GATGACCT GG | GCCAGGTC ATCAATTG TCTTTTCCA G |
| PAX8-PPARG | 28 | GCCACACCC CCTACTCCTC CTACAGC | CCAGAATGG CATCTCTGT G | GCCACACCC CCTACTCCT CCTACAGC | CCAGAATGGC ATCTCTGTG | GCCACACCC CCTACTCCTC CTACAGC | GCCAGAATG GCATCTCTGT G | GCCACACC CCCTACTC CTCCTACA GC | GCCAGAAT GGCATCTC TGTG |
| | 29 | CCTCCGTGT ACGGGCAGT TCACGGGCC A | AGAATGGCA TCTCTGTGTC AACCA | CCTCCGTGT ACGGGCAGT TCACGGGCC A | AGAATGGCAT CTCTGTGTCA ACCA | GCCTCCGTG TACGGGCAG TTCACGGGC CA | GAGAATGGC ATCTCTGTGT CAACCA | GCCTCCGT GTACGGGC AGTTCACG GGCCA | GAGAATGG CATCTCTG TGTCAACC A |
| | 30 | GCCTCCCCC AGCCACACC AAAGGCGAG | TGGTGGGCC AGAATGGCA TCTC | GCCTCCCCC AGCCACACC AAAGGCGAG | TGGTGGGCCA GAATGGCATC TC | GCCTCCCCC AGCCACACC AAAGGCGAG | GTGGTGGGC CAGAATGGC ATCTC | GCCTCCCC CAGCCACA CCAAAGGC GAG | GTGGTGGG CCAGAATG GCATCTC |
| | 31 | CCTCCTCTGC CATCGCAGG CATGGTG | CCAGAATGG CATCTCTGT GTCA | CCTCCTCTG CCATCGCAG GCATGGTG | CCAGAATGGC ATCTCTGTGT CA | GCCTCCTCT GCCATCGCA GGCATGGTG | GCCAGAATG GCATCTCTGT GTCA | GCCTCCTC TGCCATCG CAGGCATG GTG | GCCAGAAT GGCATCTC TGTGTCA |
| ETV6-NTRK 3 | 32 | GTCTCTGTCT CCCCGCCTG A | CACGATGTC TCTCCTCTTA ATG | GTCTCTGTCT CCCCGCCTG A | CACGATGTCT CTCCTCTTAA TG | GTCTCTGTCT CCCCGCCTG A | GCACGATGTC TCTCCTCTTA ATG | GTCTCTGT CTCCCCGC CTGA | GCACGATG TCTCTCCTC TTAATG |
| | 33 | CCGGAGGTC ATACTGCAT CAGA | TTGATGTGG TGCAGTGGG | CCGGAGGTC ATACTGCAT CAGA | TTGATGTGGT GCAGTGGG | GCCGGAGGT CATACTGCA TCAGA | GTTGATGTGG TGCAGTGGG | GCCGGAGG TCATACTG CATCAGA | GTTGATGT GGTGCAGT GGG |

**Table 20 Primers for pathogen detection**

| Pathogenic microorganis m | Ordinary primer | | With phosphorylated 5' end | | With G added to 5' end | | With G added to 5' end and phosphorylated | |
|---|---|---|---|---|---|---|---|---|
| | F | R | F | R | F | R | F | R |
| Influenza A virus | AATGGCTA AAGACAAG ACCA | GACAAAGCG TCTACGCTGC AG | AATGGCTAA AGACAAGA CCA | GACAAAGCG TCTACGCTG CAG | GAATGGCTAA AGACAAGACC A | GACAAAGC GTCTACGC TGCAG | GAATGGCT AAAGACAA GACCA | GACAAAGC GTCTACGC TGCAG |
| HINI | ATTATGAG GAGCTAAG AGAG | ACATGCTGCC GTTACACC | ATTATGAGG AGCTAAGAG AG | ACATGCTGC CGTTACACC | GATTATGAGG AGCTAAGAGA G | GACATGCT GCCGTTAC ACC | GATTATGA GGAGCTAA GAGAG | GACATGCT GCCGTTAC ACC |
| H3N2 | GAGAAAAC TGCACACTA ATAGATG | TAACAGTTGC TGTAGGCTTT | GAGAAAACT GCACACTAA TAGATG | TAACAGTTG CTGTAGGCT TT | GAGAAAACTG CACACTAATAG ATG | GTAACAGT TGCTGTAG GCTTT | GAGAAAAC TGCACACTA ATAGATG | GTAACAGT TGCTGTAG GCTTT |
| H7N9 | AAAATGTC CGAGATAT GT | CTATAGCACC AAATAGGCC TC | AAAATGTCC GAGATATGT | CTATAGCAC CAAATAGGC CTC | GAAAATGTCC GAGATATGT | GCTATAGC ACCAAATA GGCCTC | GAAAATGT CCGAGATA TGT | GCTATAGC ACCAAATA GGCCTC |
| influenza B virus | TAAGATGT GGCGAATG CA | GTGTCTTGAG AAAATACCA | TAAGATGTG GCGAATGCA | GTGTCTTGA GAAAATACC A | GTAAGATGTG GCGAATGCA | GTGTCTTG AGAAAATA CCA | GTAAGATG TGGCGAAT GCA | GTGTCTTG AGAAAATA CCA |
| Influenza C virus | CTTCTGCTT GCAATCTA AA | CTAATCCCAA AGAGGCTAA TG | CTTCTGCTT GCAATCTAA A | CTAATCCCA AAGAGGCTA ATG | GCTTCTGCTTG CAATCTAAA | GCTAATCC CAAAGAGG CTAATG | GCTTCTGCT TGCAATCTA AA | GCTAATCC CAAAGAGG CTAATG |
| Human parainfluenza virus 1 | AGTTATGCT CCTTGCCCA C | GATGGTCAA AAGTTATATC TTC | AGTTATGCT CCTTGCCCA C | GATGGTCAA AAGTTATAT CTTC | GAGTTATGCTC CTTGCCCAC | GATGGTCA AAAGTTAT ATCTTC | GAGTTATG CTCCTTGCC CAC | GATGGTCA AAAGTTAT ATCTTC |
| Human Parainfluenza virus 2 | TCAAAGTCT TCGAGTGGT GTA | TCCCTTTAAG AGCTCAATG AT | TCAAAGTCT TCGAGTGGT GTA | TCCCTTTAA GAGCTCAAT GAT | GTCAAAGTCTT CGAGTGGTGTA | GTCCCTTTA AGAGCTCA ATGAT | GTCAAAGT CTTCGAGTG GTGTA | GTCCCTTTA AGAGCTCA ATGAT |
| Human Parainfluenza virus 3 | CAAAATCA TTAATTCGG GTTGG | ATCTTCATAT CTGATTTTAT CAC | CAAAATCAT TAATTCGGG TTGG | ATCTTCATA TCTGATTTT ATCAC | GCAAAATCATT AATTCGGGTTG G | GATCTTCA TATCTGATT TTATCAC | GCAAAATC ATTAATTCG GGTTGG | GATCTTCA TATCTGATT TTATCAC |
| Human Parainfluenza virus 4 | TTAACGGA AGAACCAC AAT | GGGACATCTT CCACATGTGC ATTTTTCG | TTAACGGAA GAACCACAA T | GGGACATCT TCCACATGT | GTTAACGGAA GAACCACAAT | GGGACATC TTCCACAT | GTTAACGG AAGAACCA CAAT | GGGACATC TTCCACAT |
| | | | | GCATTTTTC G | | GTGCATTTTT TCG | | GTGCATTTTT TCG |
| Respiratory syncytial virus | CAAATATG GAAACATA CGTGAAC | GCACCCATAT TGTAAGTGAT GC | CAAATATGG AAACATACG TGAAC | GCACCCATA TTGTAAGTG ATGC | GCAAATATGG AAACATACGTG AAC | GCACCCAT ATTGTAAG TGATGC | GCAAATAT GGAAACAT ACGTGAAC | GCACCCAT ATTGTAAG TGATGC |
| Human coronavirus 229E | GGGATCAA TTCCTTTAC | TATACACATT CACCGTTATA | GGGATCAAT TCCTTTAC | TATACACAT TCACCGTTA TA | GGGATCAATTC CTTTAC | GTATACAC ATTCACCG TTATA | GGGATCAA TTCCTTTAC | GTATACAC ATTCACCG TTATA |
| Human coronavirus HKU1 | TGTCGCAA GTTTGGCA | TATTACCATA AGTAGTAAA AC | TGTCGCAAG TTTGGCA | TATTACCAT AAGTAGTAA AAC | GTGTCGCAAGT TTGGCA | GTATTACC ATAAGTAG TAAAAC | GTGTCGCA AGTTTGGCA | GTATTACC ATAAGTAG TAAAAC |
| human coronavirus oc43 | ATTGCACCA TAGCTCAAC TC | TATTTCAAGT CTAGCCGGTG AT | ATTGCACCA TAGCTCAAC TC | TATTTCAAG TCTAGCCGG TGAT | GATTGCACCAT AGCTCAACTC | GTATTTCA AGTCTAGC CGGTGAT | GATTGCAC CATAGCTCA ACTC | GTATTTCA AGTCTAGC CGGTGAT |
| Human coronavirus NL63 | GTTTTGTCA ATTTTAATG TG | TTAGTTTCAG GATTAAAAG C | GTTTTGTCA ATTTTAATG TG | TTAGTTTCA GGATTAAAA GC | GTTTTGTCAAT TTTAATGTG | GTTAGTTTC AGGATTAA AAGC | GTTTTGTCA ATTTTAATG TG | GTTAGTTTC AGGATTAA AAGC |
| MERS coronavirus | CTTATGTTA TGCACAACT ATG | AGCTCTAACT TCTTTGTAGA A | CTTATGTTA TGCACAACT ATG | AGCTCTAAC TTCTTTGTA GAA | GCTTATGTTAT GCACAACTATG | GAGCTCTA ACTTCTTTG TAGAA | GCTTATGTT ATGCACAA CTATG | GAGCTCTA ACTTCTTTG TAGAA |
| SARS coronavirus | GGCTTTGTT GGAAGTGC AA | ACGACAATT GTATCTGTGA | GGCTTTGTT GGAAGTGCA A | ACGACAATT GTATCTGTG A | GGCTTTGTTGG AAGTGCAA | GACGACAA TTGTATCTG TGA | GGCTTTGTT GGAAGTGC AA | GACGACAA TTGTATCTG TGA |
| covid-19 | GTCTGCGGT ATGTGGAA AGG | TGCCTGTGCC GCACGGTGT AAGAC | GTCTGCGGT ATGTGGAAA GG | TGCCTGTGC CGCACGGTG TAAGAC | GTCTGCGGTAT GTGGAAAGG | GTGCCTGT GCCGCACG GTGTAAGA C | GTCTGCGGT ATGTGGAA AGG | GTGCCTGT GCCGCACG GTGTAAGA C |

**Table 21 TA cloning primers**

| Primer type | F | R |
|---|---|---|
| ordinary primer | ATGCCCTTCCAGCCAACTCATATACT | AGGCACGTACCGTTTGCACCATATT |
| Primers with phosphorylated 5' end | ATGCCCTTCCAGCCAACTCATATACT | AGGCACGTACCGTTTGCACCATATT |
| Primers with G added to 5' end | GATGCCCTTCCAGCCAACTCATATACT | GAGGCACGTACCGTTTGCACCATATT |
| Primers with G added to 5' end and phosphorylated | GATGCCCTTCCAGCCAACTCATATACT | GAGGCACGTACCGTTTGCACCATATT |

Each of the technical features of the above-mentioned embodiments may be combined arbitrarily. To simplify the description, not all the possible combinations of each of the technical features in the above embodiments are described. However, all of the combinations of these technical features should be considered as within the scope of this application, as long as such combinations do not contradict with each other.

The aforementioned embodiments only illustrate several embodiments of the present application, which facilitate a specific and detailed understanding of the technical solutions of the present application, but they cannot be understood to limit the protection scope of the present application. It should be noted that a plurality of variations and modifications may be made by those skilled in the art without departing from the conception of the present application, which are all within the scope of protection of the present application. Accordingly, the scope of protection of the present application shall be based on the appended claims, and the description may be used to interpret the content of the claims.

## Claims

1. An amplification primer for sequencing library construction comprising a primer sequence fragment complementary to a target fragment and a base G ligated to the 5' end of the primer sequence fragment, wherein the amplification primer and the target fragment are not complementary at the base G.

2. The amplification primer according to claim 1, wherein the base G is directly ligated to the 5' end of the primer sequence fragment.

3. The amplification primer according to any one of claims 1 to 2, wherein the base G is a base modified by phosphorylation.

4. Use of the amplification primer according to any one of claims 1 to 3 in preparation of a kit for constructing a sequencing library.

5. A method for constructing a sequencing library, comprising:
providing a primer sequence fragment used for performing amplification and library construction on a deoxyribonucleic acid, the primer sequence fragment being complementary to a target fragment,
performing a treatment on the primer sequence fragment to obtain an amplification primer with a base G at the 5' end, wherein the treatment includes:
ligating a base G to the 5' end of the primer sequence fragment if the base at the 5' end of the primer sequence fragment is not G, or
ligating or not ligating a base G to the 5' end of the primer sequence fragment if the base at the 5' end of the primer sequence fragment is G;
performing cyclic amplification on the deoxyribonucleic acid using the amplification primer, to obtain an amplification fragment with a base C at the 3' end;
adding a base A to the 3' end of the amplification fragment; and
ligating an adaptor containing a T sticky end to the amplification fragment with base A added to the 3' end, wherein the adaptor contains a sequence required by a sequencing platform.

6. The method for constructing a sequencing library according to claim 5, further comprising: phosphorylating the base G at the 5' end of the amplification primer before performing amplification on the deoxyribonucleic acid.

7. The method for constructing a sequencing library according to claim 5 or 6, wherein the deoxyribonucleic acid is selected from the group consisting of a sample DNA, a sample plasmid, and a deoxyribonucleic acid obtained by reverse transcription of a sample RNA.

8. A sequencing library constructed by the method for constructing a sequencing library according to any one of claims 5 to 7.

9. A kit for constructing a sequencing library, comprising the amplification primer according to any one of claims 1 to 3.

10. The kit according to claim 9, further comprising at least one of a reagent for adding base A, a reagent for adding adaptors, a reagent for PCR amplification and a reagent for purification.

11. A sequencing method, comprising: constructing a sequencing library for a sample using the method for constructing a sequencing library according to any one of claims 5 to 7, and performing sequencing.

12. The sequencing method according to claim 11, wherein the sequencing method is used to perform on the sample any one of mutation site detection, chromosome ploidy detection, gene fusion detection, and pathogenic microorganism detection.

13. The sequencing method according to claim 12, wherein the mutation site detection comprises at least one of intestinal cancer mutation sites, cervical cancer mutation sites, and urothelial cancer mutation sites.

14. The sequencing method according to claim 12, wherein the chromosome ploidy comprises at least one of the chromosome ploidy of intestinal cancer, the chromosome ploidy of cervical cancer, and the chromosome ploidy of urothelial cancer.

15. The sequencing method according to claim 12, wherein the gene fusion comprises at least one of EML4-ALK gene fusion, CD74- ROSI gene fusion, CCDC6-RET gene fusion, NCOA4-RET gene fusion, and TPM3-NTRK1 gene fusion.

16. The sequencing method according to claim 12, wherein the pathogenic microorganism comprises at least one of influenza A virus, influenza B virus and SARS-CoV-2.

17. The sequencing method according to any one of claims 11 to 16, wherein the sequencing is high-throughput sequencing.

18. A mutation site detection kit, comprising the amplification primer according to any one of claims 1 to 3.

19. A chromosome ploidy detection kit, comprising the amplification primer according to any one of claims 1 to 3.

20. A gene fusion detection kit, comprising the amplification primer according to any one of claims 1 to 3.

21. A pathogenic microorganism detection kit, comprising the amplification primer according to any one of claims 1 to 3.

22. A TA cloning method, comprising the steps: performing PCR amplification using the amplification primer according to any one of claims 1 to 3 to add A to the end of an amplification product.
